# EUROPEAN PATENT APPLICATION

(11) **EP 3 808 745 A1**
(43) Date of publication of application: **21.04.2021**
(21) Application number: 19807382.7
(22) Date of filing: 24.05.2019
(51) Int. Cl.: C07D 471/04, A61K 31/395, A61P 31/12, A61P 35/00

(54) **CRYSTAL FORM OF HYDROCHLORIDE OF PYRAZOLOHETEROARYL DERIVATIVE AND PREPARATION METHOD**

(30) Priority: 25.05.2018 CN 201810512563
(71) Applicant: Jiangsu Hengrui Medicine Co., Ltd., Lianyungang, Jiangsu 222047 (CN); Shanghai Hengrui Pharmaceutical Co., Ltd, Shanghai 200245 (CN)
(72) Inventor: CAO, Xiaoli, Lianyungang, Jiangsu 222047 (CN); DU, Zhenxing, Lianyungang, Jiangsu 222047 (CN); WANG, Likun, Lianyungang, Jiangsu 222047 (CN)
(74) Representative: dompatent von Kreisler Selting Werner - Partnerschaft von Patent- und Rechtsanwälten mbB
(86) International application number: PCT/CN2019/088250
(87) International publication number: WO 2019/223773

(57) **Abstract**

The present invention relates to a crystal form of a hydrochloride of a pyrazoloheteroaryl derivative and a preparation method. In particular, the present invention relates to crystal forms I and II of a dihydrochloride of a compound represented by formula (I), crystal forms A , B and C of a monohydrochloride of the compound represented by formula (I), and a preparation method thereof. The crystal forms of the compound represented by formula (I) of the present invention have good crystal stability and can be better used for clinical treatment.

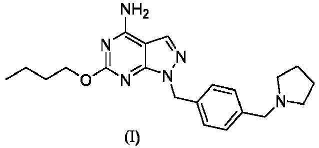

## Description

The present application claims the benefit of Chinese Patent Application No. CN201810512563.7 filed on May 25, 2018, the contents of which are incorporated herein by reference in their entireties.

### Technical Field

The present disclosure relates to a crystal form I and a crystal form II of 6-butoxy-1-(4-(pyrrolidin-1-ylmethyl)benzyl)-1*H*-pyrazolo[3,4-*d*]pyrimidin-4-amine dihydrochloride and a crystal form A, a crystal form B, and a crystal form C of 6-butoxy-1-(4-(pyrrolidin-1-ylmethyl)benzyl)-1*H*-pyrazolo[3,4-*d*]pyrimidin-4-amine monohydrochloride, and preparation methods thereof.

### Background

Toll-like receptors (TLRs) are a class of important protein molecules involved in innate immunity. TLRs are single, membrane-spanning, non-catalytic receptors, usually expressed on sentinel cells such as macrophages and dendritic cells, and can recognize structurally conserved molecules produced by microbes. Once these microbes have broken through physical barriers such as skin or intestinal tract mucosa, they are recognized by TLRs, thereby activating immune cell responses (Mahla, RS. et al., Front Immunol. 4: 248 (2013)). The ability of immune system to broadly recognize pathogenic microorganisms is, in part, due to the widespread presence of toll-like immunoreceptors (TLRs).

There are at least ten different TLRs in mammals. Ligands and corresponding signaling cascades have been identified for some of these receptors. TLR7 is a member of the subgroup of TLRs (TLRs 3, 7, 8, and 9), localized in the endosomal compartment of cells which are specialized to detect non-self nucleic acids. TLR7 plays a key role in anti-viral defence via the recognition of ssRNA (Diebold S. S. et al., Science, 2004: 303, 1529-1531; and Lund J. M. et al., PNAS, 2004: 101, 5598-5603). TLR7 has a restricted expression-profile in human, and is expressed predominantly by B cells and plasmacytoid dendritic cells (pDC), and to a lesser extent by monocytes. Plasmacytoid DCs are a unique population of lymphoid-derived dendritic cells (0.2-0.8% of peripheral blood mononuclear cells (PBMCs)), which are the primary type I interferon-producing cells secreting high levels of interferon-alpha (IFNα) and interferon-beta (IFNβ) in response to viral infections (Liu Y-J, Annu. Rev. Immunol., 2005: 23, 275-306).

Many diseases and disorders are related to abnormalities in TLRs, such as melanoma, non-small cell lung cancer, hepatocellular carcinoma, basal cell carcinoma, renal cell carcinoma, myeloma, allergic rhinitis, asthma, chronic obstructive pulmonary disease (COPD), ulcerative colitis, hepatic fibrosis, and viral infections such as HBV, Flaviviridae viruses, HCV, HPV, RSV, SARS, HIV, or influenza viruses infections. Therefore, the use of TLR agonists to treat related diseases is very promising.

Since TLR7 and TLR8 are highly homologous, the ligand of TLR7 in most cases is also that of TLR8. TLR8 stimulation mainly induces the production of cytokines such as tumor necrosis factor α (TNF-α) and chemokine. Interferon α is one of the main drugs for treating chronic hepatitis B or hepatitis C, while TNF-α is a pro-inflammatory cytokine, and its over-secretion may cause severe side effects. Therefore, the selectivity for TLR7 and TLR8 is critical for the development of TLR7 agonists for treating virus infection diseases.

A TLR7 agonist is provided in the application with application number PCT/CN2017/113007 (filling date: November 27, 2017), and its formula is as follows:

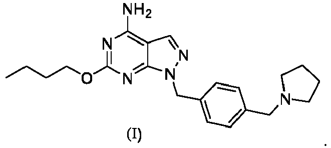

There are currently patent applications related to TLR7 agonists, such as WO2005025583, WO2007093901, WO2008011406, WO2009091032, WO2010077613, WO2010133882, WO2011031965, WO2012080730, *etc.*

The crystal form structure of active pharmaceutical ingredients often affects the chemical stability of the drug. Different crystallization conditions and storage conditions may lead to changes in the crystal form structure of compounds, sometimes accompanied by the formation of other crystal forms. Generally speaking, amorphous drug products have no regular crystal structure and often have other drawbacks, such as poor product stability, finer precipitate, difficult filtration, easy agglomeration, and poor fluidity. Polymorphs of drugs have different requirements for product storage, production and scale-up. Therefore, it is necessary for in-depth study of the crystal form of the compound of formula (I) and related preparation methods to improve various properties of the compound of formula (I).

### Content of the present invention

The present disclosure provides a hydrochloride salt of the compound represented by formula (I),

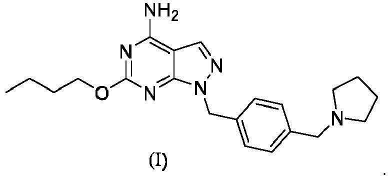

The present disclosure provides a dihydrochloride salt of the compound represented by formula (I).

The present disclosure provides a crystal form I and a crystal form II of a dihydrochloride salt of the compound represented by formula (I), a crystal form A, a crystal form B, and a crystal form C of a monohydrochloride salt of the compound represented by formula (I), and preparation methods thereof, the crystal forms of the compound of formula (I) of the present disclosure have good crystal form stability.

One aspect of the present disclosure provides a crystal form I of a dihydrochloride salt of the compound represented by formula (I), wherein the X-ray powder diffraction pattern thereof has characteristic peaks at 2θ angles of 7.182, 8.520, 12.275, 15.057, 15.614, 20.994, 21.804, and 22.934.

In a preferred embodiment, the present disclosure provides a crystal form I of a dihydrochloride salt of the compound represented by formula (I), wherein the X-ray powder diffraction pattern thereof has characteristic peaks at 2θ angles of 7.182, 8.520, 11.152, 12.275, 15.057, 15.614, 15.902, 17.162, 20.384, 20.994, 21.804, 22.934, 24.360, 26.260, 26.630, 27.209, and 29.724.

In a more preferred embodiment, the present disclosure provides a crystal form I of a dihydrochloride salt of the compound represented by formula (I), wherein the X-ray powder diffraction pattern thereof has characteristic peaks at 2θ angles of 7.182, 7.722, 8.520, 11.152, 12.275, 15.057, 15.614, 15.902, 17.162, 20.384, 20.994, 21.804, 22.934, 24.360, 25.320, 26.260, 26.630, 27.209, 27.920, 29.724, 30.720, and 32.270.

One aspect of the present disclosure provides a crystal form II of a dihydrochloride salt of the compound represented by formula (I), wherein the X-ray powder diffraction pattern thereof has characteristic peaks at 2θ angles of 9.999, 10.801, 12.461, 15.761, 17.020, 18.680, 20.558, 20.863, 24.541, 26.240, and 26.660.

In a preferred embodiment, the present disclosure provides a crystal form II of the compound represented by formula (I), wherein the X-ray powder diffraction pattern thereof has characteristic peaks at 2θ angles of 8.479, 9.999, 10.801, 12.461, 13.725, 14.120, 15.761, 17.020, 18.680, 20.135, 20.558, 20.863, 21.641, 22.960, 24.202, 24.541, 26.240, 26.660, 28.262, and 28.681.

In a more preferred embodiment, the present disclosure provides a crystal form II of the compound represented by formula (I), wherein the X-ray powder diffraction pattern thereof has characteristic peaks at 2θ angles of 5.002, 7.202, 8.479, 9.999, 10.801, 11.220, 11.995, 12.461, 13.725, 14.120, 15.761, 16.484, 17.020, 18.680, 20.135, 20.558, 20.863, 21.289, 21.641, 22.319, 22.960, 24.202, 24.541, 26.240, 26.660, 27.196, 28.262, 28.681, 29.518, 31.017, 31.355, 32.725, 33.198, 36.810, 37.880, 39.335, and 41.004.

The present disclosure provides a monohydrochloride salt of the compound represented by formula (I).

Another aspect of the present disclosure provides a crystal form A of a monohydrochloride salt of the compound represented by formula (I), wherein the X-ray powder diffraction pattern thereof has characteristic peaks at 2θ angles of 9.647, 13.306, 13.644, 14.936, 17.533, 18.866, 20.261, and 22.515.

In a more preferred embodiment, the present disclosure provides a crystal form A of a monohydrochloride salt of the compound represented by formula (I), wherein the X-ray powder diffraction pattern thereof has characteristic peaks at 2θ angles of 9.647, 13.018, 13.306, 13.644, 14.936, 17.533, 18.866, 20.261, 20.836, 21.038, 21.684, 22.515, 24.775, 25.396, 26.306, 27.095, 28.182, 28.742, 29.621, and 30.388.

Another aspect of the present disclosure provides a crystal form B of a monohydrochloride salt of the compound represented by formula (I), wherein the X-ray powder diffraction pattern thereof has characteristic peaks at 2θ angles of 12.421, 13.937, 17.095, 17.492, 18.647, 19.317, 21.823, 22.183, and 26.321.

In a more preferred embodiment, the present disclosure provides a crystal form B of a monohydrochloride salt of the compound represented by formula (I), wherein the X-ray powder diffraction pattern thereof has characteristic peaks at 2θ angles of 7.094, 12.421, 13.937, 14.900, 15.837, 17.095, 17.492, 18.647, 19.317, 21.823, 22.183, 23.777, 24.391, 26.321, 26.857, 27.432, 29.918, and 30.946.

Another aspect of the present disclosure provides a crystal form C of a monohydrochloride salt of the compound represented by formula (I), wherein the X-ray powder diffraction pattern thereof has characteristic peaks at 2θ angles of 9.641, 10.199, 12.176, 15.950, 17.288, 18.579, 19.859, 20.675, 21.083, 21.838 and 24.628.

In a more preferred embodiment, the present disclosure provides a crystal form C of a monohydrochloride salt of the compound represented by formula (I), wherein the X-ray powder diffraction pattern thereof has characteristic peaks at 2θ angles of 9.641, 10.199, 12.176, 12.542, 13.302, 15.118, 15.592, 15.950, 17.288, 18.579, 19.547, 19.859, 20.675, 21.083, 21.838, 23.795, 23.963, 24.628, 25.222, 26.914, 28.068, 28.886, and 30.179.

The present disclosure provides a preparation method of a hydrochloride salt of the compound represented by formula (I), comprising a step of salifying the compound represented by formula (I) with hydrochloric acid.

The present disclosure further provides a preparation method of a crystal form I of a dihydrochloride salt of the compound represented by formula (I), wherein the method is selected from:

a method i: placing the compound represented by formula (I) in a solvent for crystallization, clarifying, adding hydrochloric acid, crystallizing, filtering, and drying to obtain the target crystal form I; or

a method ii: placing the dihydrochloride salt of the compound represented by formula (I) in a solvent for crystallization, crystallizing, filtering, and drying to obtain the target crystal form I, wherein the crystallizing method is selected from crystallizing at room temperature, crystallizing by cooling, crystallizing by volatilizing solvent, or crystallizing by adding a seed crystal to induce crystallization;

in the method i or the method ii, the solvent for crystallization does not include a mixed solvent of isopropanol-tetrahydrofuran;

in the method i or the method ii, the solvent for crystallization is one or more selected from ether solvents, alcohol solvents, ester solvents, ketone solvents, nitrile solvents, and halogenated hydrocarbon solvents;

in the method i or the method ii, the ether solvent includes, but not limited to, tetrahydrofuran, diethyl ether, propylene glycol monomethyl ether, methyl *tert*-butyl ether, isopropyl ether or 1,4-dioxane;

in the method i or the method ii, the alcohol solvent includes, but not limited to, methanol, ethanol, isopropanol, *n*-propanol, isopentanol or trifluoroethanol;

in the method i or the method ii, the ester solvent includes, but not limited to, ethyl acetate, isopropyl acetate or butyl acetate;

in the method i or the method ii, the ketone solvent includes, but not limited to, acetone, acetophenone, methyl isobutyl ketone or methyl pyrrolidone;

in the method i or the method ii, the nitrile solvent includes, but not limited to, acetonitrile or propionitrile;

in the method i or the method ii, the halogenated hydrocarbon solvent includes, but not limited to, chloromethane, dichloromethane, chloroform or carbon tetrachloride;

in the method i or the method ii, the amount of the hydrochloric acid is 2-30 times, preferably 2-15 times, and most preferably 2-5 times the amount of substance of the compound represented by formula (I).

In the preparation method of the crystal form I of a dihydrochloride salt of the compound represented by formula (I) of the present disclosure, when the solvent for crystallization is a mixed solvent, the mixed solvent is not isopropanol-tetrahydrofuran, and includes, but not limited to, isopropanol-isopropyl acetate, isopropanol-isopropyl ether, isopropanol-dioxane, ethanol-dioxane, ethanol-tetrahydrofuran, ethanol-isopropyl ether, ethanol-isopropyl acetate, ethanol-acetonitrile, isopropanol-acetonitrile, methanol-isopropyl ether, methanol-isopropyl acetate, methanol-acetonitrile, dichloromethane-tetrahydrofuran, isopropanol-tetrahydrofuran, isopropanol-ethyl acetate or methanol-ethyl acetate.

The present disclosure further provides a preparation method of a crystal form II of the compound represented by formula (I), comprising placing the compound of formula (I) in a solvent for crystallization, clarifying, adding hydrochloric acid, crystallizing, filtering, and drying to obtain the target crystal form II,

the solvent for crystallization is a mixed solvent of isopropanol-tetrahydrofuran; the amount of hydrochloric acid is 2-30 times, preferably 2-15 times, most preferably 2-5 times the amount of substance of the compound represented by formula (I).

The present disclosure further provides a preparation method of a crystal form A of the compound represented by formula (I), the method is selected from:

a method i: placing the compound represented by formula (I) in a solvent for crystallization, clarifying, adding hydrochloric acid, crystallizing, filtering, and drying to obtain the target crystal form A; or

a method ii: placing a monohydrochloride salt of the compound represented by formula (I) in a solvent for crystallization, crystallizing, filtering, and drying to obtain the target crystal form A, wherein the crystallizing method is selected from crystallizing at room temperature, crystallizing by cooling, crystallizing by volatilizing solvent, or crystallizing by adding a seed crystal to induce crystallization;

in the method i or the method ii, the solvent for crystallization is at least one selected from nitrile solvents and ketone solvents;

in the method i or the method ii, the ketone solvent is selected from acetone, acetophenone, methyl isobutyl ketone or methyl pyrrolidone, preferably acetone;

in the method i or the method ii, the nitrile solvent is selected from acetonitrile or propionitrile, preferably acetonitrile;

in the method i or the method ii, the amount of the hydrochloric acid is 1-2 times (excluding 2 times) the amount of substance of the compound represented by formula (I).

The present disclosure further provides a preparation method of a crystal form B of the compound represented by formula (I), the method is selected from:

a method i: placing the compound represented by formula (I) in a solvent for crystallization, clarifying, adding hydrochloric acid, crystallizing, filtering, and drying to obtain the target crystal form B; or

a method ii: placing a monohydrochloride salt of the compound represented by formula (I) in a solvent for crystallization, crystallizing, filtering, and drying to obtain the target crystal form B, wherein the crystallizing method is selected from crystallizing at room temperature, crystallizing by cooling, crystallizing by volatilizing solvent, or crystallizing by adding a seed crystal to induce crystallization;

in the method i or the method ii, the solvent for crystallization is selected from ester solvents, the ester solvent is selected from ethyl acetate, isopropyl acetate or butyl acetate, preferably ethyl acetate;

in the method i or the method ii, the amount of the hydrochloric acid is 1-2 times (excluding 2 times) the amount of the compound represented by formula (I).

The present disclosure further provides a preparation method of a crystal form C of a monohydrochloride salt of the compound represented by formula (I), the method is selected from:

a method i: placing the compound represented by formula (I) in a solvent for crystallization, clarifying, adding hydrochloric acid, crystallizing, filtering, and drying to obtain the target crystal form C; or

a method ii: placing a monohydrochloride salt of the compound represented by formula (I) in a solvent for crystallization, crystallizing, filtering, and drying to obtain the target crystal form C, wherein the crystallizing method is selected from crystallizing at room temperature, crystallizing by cooling, crystallizing by volatilizing solvent, or crystallizing by adding a seed crystal to induce crystallization;

in the method i or the method ii, the solvent for crystallization is selected from ether solvents, the ether solvent is selected from tetrahydrofuran, diethyl ether, propylene glycol monomethyl ether, methyl *tert*-butyl ether, isopropyl ether or 1,4-dioxane, preferably 1,4-dioxane; the amount of the hydrochloric acid is 1-2 times (excluding 2 times) the amount of substance of the compound represented by formula (I).

In the preparation methods of the crystal form I and crystal form II of the dihydrochloride salt of the compound represented by formula (I), and the crystal form A, crystal form B, and crystal form C of the monohydrochloride salt of the compound represented by formula (I), the temperature at which the compound represented by formula (I) is clarified in the solvent for crystallization and the hydrochloric acid is added is not specifically defined, the reaction temperature can change with the change of the solvent, and specific reaction temperature can be -20 °C to 100 °C, preferably 0 °C to 80 °C, more preferably 15 °C to 60 °C, when hearting is carried out, the crystallizing method may be crystallizing by cooling.

The hydrochloric acid involved in the preparation method of the hydrochloride salts (including the preparation method of the hydrochloride salts and the crystal forms) of the present disclosure can be concentrated hydrochloric acid, hydrogen chloride gas, or a solution of hydrogen chloride gas in the solvent for crystallization, or concentrated hydrochloric acid diluted with the solvent for crystallization.

The crystal forms of the hydrochloride salts of the compound represented by formula (I) provided in the present disclosure optionally contain stoichiometric water or non-stoichiometric water, once the peak positions the XPRD patterns are the same as that of each crystal form of the present disclosure, it falls within the protection scope of the present disclosure.

The present disclosure also relates to a pharmaceutical composition comprising the hydrochloride salt of the compound represented by formula (I), the crystal form I, the crystal form II of the dihydrochloride salt of the compound represented by formula (I), the crystal form A, the crystal form B, the crystal form C of the monohydrochloride salt of the compound represented by formula (I), and optionally one or more pharmaceutical carriers and/or diluents. The pharmaceutical composition can be made into any pharmaceutically acceptable preparation. For example, the hydrochloride salt of the compound represented by formula (I), the crystal form I, the crystal form II of the dihydrochloride salt of the compound represented by formula (I), the crystal form A, the crystal form B, the crystal form C of the monohydrochloride salt of the compound represented by formula (I), or the pharmaceutical preparation can be formulated as tablets, capsules, pills, granules, solutions, suspensions, syrups, injections (including injections, sterile powders for injection, and concentrated solutions for injection), suppositories, inhalants or sprays.

In addition, the pharmaceutical composition of the present disclosure can also be administered to patients or subjects in need of such treatment in any suitable way of administration, such as oral, parenteral, rectal, pulmonary or topical administration. When used for oral administration, the pharmaceutical composition can be made into oral preparations, such as oral solid preparations, such as tablets, capsules, pills, granules, *etc.;* or, oral liquid preparations, such as oral solutions, oral suspensions, syrups, *etc.* When made into oral preparations, the pharmaceutical preparations may also contain suitable fillers, binders, disintegrants, lubricants and the like. When used for parenteral administration, the pharmaceutical preparations can be made into injections, including solutions for injection, sterile powders for injection, and concentrated solutions for injection. When made into injections, the pharmaceutical composition can be produced by using conventional methods existing in the pharmaceutical field. When preparing injections, the pharmaceutical preparations may not be added with additives, or appropriate additives may be added according to the nature of the drug. When used for rectal administration, the pharmaceutical preparation can be made into suppositories and the like. When used for pulmonary administration, the pharmaceutical preparations can be made into inhalants or sprays. In some preferred embodiments, the hydrochloride salt of the compound represented by formula (I), the crystal form I, the crystal form II of the dihydrochloride salt of the compound represented by formula (I), the crystal form A, the crystal form B, the crystal form C of the monohydrochloride of the compound represented by formula (I) of the present disclosure are present in the pharmaceutical composition or medicament in a therapeutically and/or prophylactically effective amount. In some preferred embodiments, the hydrochloride salt of the compound represented by formula (I), the crystal form I, the crystal form II of the dihydrochloride salt of the compound represented by formula (I), the crystal form A, the crystal form B, the crystal form C of the monohydrochloride of the compound represented by formula (I) of the present disclosure are present in the pharmaceutical composition or medicament in the form of a unit dose.

The present disclosure further relates to a preparation method of the pharmaceutical composition, comprises the following step: mixing one or more crystal forms selected from the hydrochloride salt of the compound represented by formula (I), the crystal form I and the crystal form II of the dihydrochloride salt of the compound represented by formula (I), and the crystal form A, the crystal form B, and the crystal form C of the monohydrochloride of the compound represented by formula (I) of the present disclosure with at least one of pharmaceutically acceptable carriers, diluents or excipients.

The present disclosure further relates to a use of the hydrochloride salt of the compound represented by formula (I), the crystal form I, the crystal form II of the dihydrochloride salt of the compound represented by formula (I), the crystal form A, the crystal form B, the crystal form C of the monohydrochloride salt of the compound represented by formula (I) in the manufacture of a medicament for treating viral infection caused by virus, the virus is selected from dengue virus, flavivirus, West Nile virus, Japanese encephalitis virus, tick-borne encephalitis virus, Kunjin virus, Murray Valley encephalitis virus, Saint Louis encephalitis virus, Omsk hemorrhagic fever virus, bovine viral diarrhea virus, Zika virus, HIV, HBV, HCV, HPV, RSV, SARS and/or influenza virus.

The present disclosure further relates to a use of the hydrochloride salt of the compound represented by formula (I), the crystal form I, the crystal form II of the dihydrochloride salt of the compound represented by formula (I), the crystal form A, the crystal form B, the crystal form C of the monohydrochloride salt of the compound represented by formula (I) in the manufacture of a medicament for treating or preventing melanoma, non-small cell lung cancer, hepatocellular carcinoma, basal cell carcinoma, renal cell carcinoma, bladder cancer, myeloma, allergic rhinitis, asthma, COPD, ulcerative colitis, and/or hepatic fibrosis.

The "heating" in the preparation method provided by the present disclosure refers to that the heating temperature does not exceed the boiling point temperature corresponding to the solvent used; the "lowering temperature", "cooling" in the preparation method provided in the present disclosure refer to the internal temperature of the system is lowered to any temperature lower than the heating temperature. The temperature can be a point value or an interval value. The "lowering temperature" and "cooling" processes can be programmed or non-programmed. In addition, it is known to those skilled in the art that stirring operation is optionally performed in the lowering temperature or cooling process.

The determination and study of the crystal forms of the compound represented by formula (I) was performed by X-ray powder diffraction pattern (XRPD) and differential scanning calorimetry (DSC).

### Detailed description of the present invention

In the description and claims of the present application, unless otherwise specified, scientific and technical terms used herein have the meanings commonly understood by those skilled in the art. However, in order to better understand the present disclosure, definitions and explanations of some relevant terms are provided below. In addition, when the definition and interpretation of the terms provided in the present application are inconsistent with the meaning commonly understood by those skilled in the art, the definition and interpretation of terms provided in the present application shall prevail.

The term "ether solvent" used in the present disclosure refers to a chain compound or a cyclic compound having an ether bond -O- and 1 to 10 carbon atoms, and specific examples include, but are not limited to, tetrahydrofuran, diethyl ether, propylene glycol monomethyl ether, methyl tert-butyl ether or 1,4-dioxane.

The term "alcohol solvent" used in the present disclosure refers to the solvent derived from substituting one or more hydrogen atoms on "C₁₋₆ alkyl" with one or more "hydroxyl" groups, the "hydroxyl" and "C₁₋₆ alkyl" are as defined above, and specific examples include, but are not limited to, methanol, ethanol, isopropanol, *n*-propanol, isopentanol or trifluoroethanol.

The term "ester solvent" used in the present disclosure refers to a combination of a lower organic acid having 1 to 4 carbon atoms and a lower alcohol having 1 to 6 carbon atoms. Its specific examples include, but are not limited to: ethyl acetate, isopropyl acetate or butyl acetate.

The term "ketone solvent" used in the present disclosure refers to a compound in which a carbonyl group (-C(O)-) is bonded to two hydrocarbon groups. Ketones can be classified into aliphatic ketones, alicyclic ketones, aromatic ketones, saturated ketones, and unsaturated ketones, depending on the hydrocarbon groups in the molecule. Its specific examples include, but are not limited to: acetone, acetophenone, methyl isobutyl ketone or methyl pyrrolidone.

The term "nitrile solvent" used in the present disclosure refers to the solvent derived from substituting one or more hydrogen atoms on "C₁₋₆ alkyl" with one or more "cyano" groups, the "cyano" and "C₁₋₆ alkyl" are as defined above, and specific examples include, but are not limited to, acetonitrile or propionitrile.

The term "aliphatic hydrocarbon solvent" used in the present disclosure refers to a hydrocarbon having the basic properties of an aliphatic compound and having 1 to 10 carbon atoms, wherein the carbon atoms in the molecule are linked to a chain-like carbon frame in which the two ends are opened and do not form a ring, for example saturated aliphatic hydrocarbon, including alkane solvents. Its specific examples include, but are not limited to: *n*-butane, *n*-pentane, *n*-hexane or *n*-heptane.

The term "halogenated hydrocarbon solvent" used in the present disclosure refers to the solvent derived from substituting one or more hydrogen atoms on "C₁₋₆ alkyl" with one or more "halogen atoms", the "halogen atom" and "C₁₋₆ alkyl" are as defined above, and specific examples include, but are not limited to, methyl chloride, dichloromethane, chloroform or carbon tetrachloride.

The "X-ray powder diffraction pattern or XRPD" used in the present disclosure refers to that according to Bragg formula 2d sin θ = nλ (in the formula, λ is the wavelength of the X-ray, λ = 1.54056 Å, the number of the diffraction order n is any positive integer, generally taking the first-order diffraction peak, n=1), when X-ray is incident to an atomic plane having d lattice plane spacing of a crystal or part of a crystal sample at a grazing angle θ (the residual angle of an incident angle, also known as Bragg angle), the Bragg equation can be then satisfied, thus this group of X-ray powder diffraction patterns can be measured.

The "X-ray powder diffraction pattern or XRPD" used in the present disclosure is obtained by using Cu-Kα radiation in X-ray Powder diffractometer.

The "differential scanning calorimetry analysis or DSC" used in the present disclosure refers to measuring the temperature difference and heat flow difference between the sample and the reference substance in the process of heating or constant temperature of the sample, in order to characterize all physical and chemical changes related to thermal effect and obtain the phase change information of the sample.

The "2θ or 2θ angle" used in the present disclosure refers to diffraction angle, θ is the Bragg angle, the unit is or degree, and the error range of 2θ is ±0.1 to ±0.5, preferably ±0.1 to ±0.3, more preferably ±0.2.

The "crystal plane spacing or crystal plane spacing (d value)" used in the present disclosure refers to 3 unit vectors a, b and c selected from the space lattice that are not parallel and connecting the two adjacent lattice points, which divide the lattice into juxtaposed parallelepiped units, that is called crystal plane spacing. The spatial lattice is divided into a set of linear lattices, called spatial lattices or lattices, according to the determined parallelepiped unit lines. The dot matrix and lattice reflect the periodicity of the crystal structure with geometric points and lines respectively, different crystal planes have different surface spacing (*i.e.,* the distance between two adjacent parallel crystal planes); the unit is Å or angstrom.

Studies have shown that the crystal form I and the crystal form II of the dihydrochloride salt of the compound represented by formula (I), the crystal form A, the crystal form B, and the crystal form C of the monohydrochloride salt of the compound represented by formula (I) have good stability and high purity, and single crystal of the crystal form I of the dihydrochloride salt of the compound represented by formula (I) is obtained; the crystal form I and the crystal form II of the dihydrochloride salt of the compound represented by formula (I), the crystal form A, the crystal form B, and the crystal form C of the monohydrochloride salt of the compound represented by formula (I) obtained in the technical solutions of the present disclosure can satisfy the pharmaceutical requirements for production, transportation and storage, have stable, repeatable and controllable production process, and can be applied to industrial production.

### Brief description of the drawings

Fig. 1 is the XPRD pattern of the crystal form I of the dihydrochloride salt of the compound represented by formula (I);

Fig. 2 is the DSC pattern of the crystal form I of the dihydrochloride salt of the compound represented by formula (I);

Fig. 3 is the TGA pattern of the crystal form I of the dihydrochloride salt of the compound represented by formula (I);

Fig. 4 is the XPRD pattern of the crystal form I of the dihydrochloride salt of the compound represented by formula (I) at DSC (150 °C);

Fig. 5 is the XPRD pattern of the crystal form I of the dihydrochloride salt of the compound represented by formula (I) at DSC (175 °C);

Fig. 6 is the DVS-first cycle pattern of the crystal form I of the dihydrochloride salt of the compound represented by formula (I);

Fig. 7 is the DVS-second cycle pattern of the crystal form I of the dihydrochloride salt of the compound represented by formula (I);

Fig. 8 is the XPRD pattern before and after DVS of the crystal form I of the dihydrochloride salt of the compound represented by formula (I);

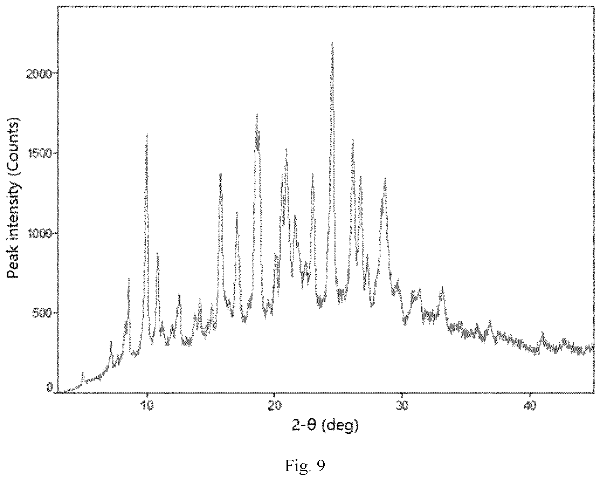

Fig. 9 is the XPRD pattern of the crystal form II of the dihydrochloride salt of the compound represented by formula (I);

Fig. 10 is the DSC pattern of the crystal form II of the dihydrochloride salt of the compound represented by formula (I);

Fig. 11 is the TGA pattern of the crystal form II of the dihydrochloride salt of the compound represented by formula (I);

Fig. 12 is the XPRD pattern of the crystal form A of the monohydrochloride salt of the compound represented by formula (I);

Fig. 13 is the DSC pattern of the crystal form A of the monohydrochloride salt of the compound represented by formula (I);

Fig. 14 is the XPRD pattern of the crystal form B of the monohydrochloride salt of the compound represented by formula (I);

Fig. 15 is the DSC pattern of the crystal form B of the monohydrochloride salt of the compound represented by formula (I);

Fig. 16 is the XPRD pattern of the crystal form C of the monohydrochloride salt of the compound represented by formula (I);

### Detailed description of the embodiment

The following examples further illustrate the present disclosure, but the present disclosure is not limited thereto.

Test conditions of the equipment used in the experiments:

The structures of the compounds are determined by nuclear magnetic resonance (NMR) or/and mass spectrometry (MS). The NMR shift (δ) is given in units of 10⁻⁶ (ppm). NMR was measured with a Bruker AVANCE-400 nuclear magnetic resonance spectrometer, the solvent was deuterated dimethyl sulfoxide (DMSO-*d*₆), deuterated chloroform (CDCl₃), deuterated methanol (CD₃OD), and the internal standard was tetramethylsilane (TMS).

The MS was measured with a FINNIGAN LCQAd (ESI) mass spectrometer (manufacturer: Thermo, model: Finnigan LCQ advantage MAX).

HPLC determination uses Agilent 1200DAD high pressure liquid chromatograph (Sunfire C18 150×4.6mm column) and Waters 2695-2996 high pressure liquid chromatograph (Gimini C18 150×4.6mm column).

XRPD is X-ray powder diffraction detection: the measurement uses Rigaku UltimaIV model combined multi-function

X-ray diffractometer, specific information collected: Cu anode (40kV, 40mA), Cu-Kα1 rays (λ line (Kα1 line, with), scanning rate: 20 scans minute, scanning range: (2q range): 3-45 scans, scanning step size: 0.02 and slit width: 0.01.

DSC is differential scanning calorimetry: TA Q2000 is used for the measurement, the heating rate is 10 °C/min, 30-300 °C, and the nitrogen purge rate is 50 mL/min.

TGA is thermogravimetric analysis: TAQ500 is used for measurement, the heating rate is 10 °C/min, the specific temperature range refers to the corresponding pattern, and the nitrogen purge rate is 60 mL/min.

DVS is dynamic vapor sorption: Surface Measurement Systems advantage 2 is used, the humidity starts from 50%, the humidity range is 0%-95%, and the step size is 10%. The judgment standard is that the mass change is less than 0.01% within 10000 min, and two cycles are performed.

The reaction progress in the embodiments are monitored by thin-layer chromatography (TLC). The developing reagent used in the reaction, the eluent system of column chromatography used in the purification of the compound and the developing reagent system of thin-layer chromatography include: A: dichloromethane/methanol system, the volume ratio of the solvents is adjusted according to the polarity of the compound, and a small amount of basic or acidic reagents such as triethylamine and acetic acid can also be added for adjustment.

### Comparative Example 1 (Preparation method in the example 1 of application of PCT/CN2017/113007)

### 6-Butoxy-1-(4-(pyrrolidin-1-ylmethyl)benzyl)-1H-pyrazolo[3,4-d]pyrimidin-4-amine

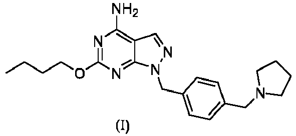

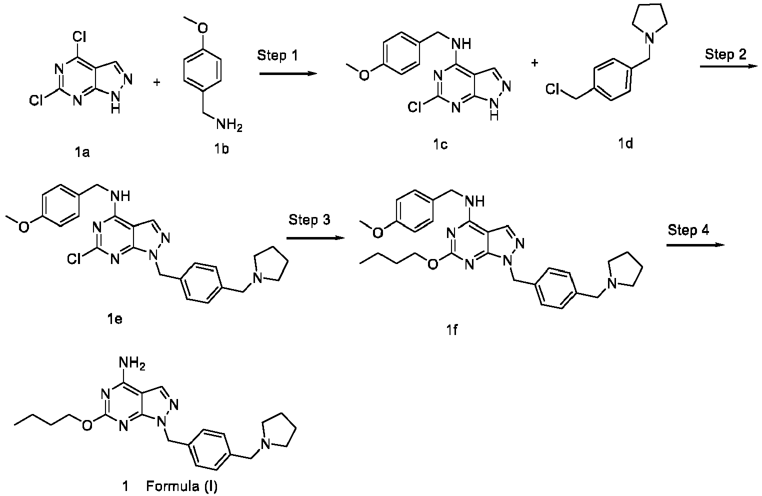

### Step 1

### 6-Chloro-N-(4-methoxybenzyl)-1H-pyrazolo[3,4-d]pyrimidin-4-amine 1c

4,6-Dichloro-1*H*-pyrazolo[3,4-*d*]pyrimidine **1a** (120 mg, 0.63 mmol), 4-methoxybenzylamine **1b** (87.1 mg, 0.63 mmol) and triethylamine (64.13 mg, 0.63 mmol) were dissolved in 2 mL of tetrahydrofuran, and the reaction solution was stirred at room temperature for 1 hour. The reaction was stopped, and the reaction solution was concentrated under reduced pressure. The residue was purified by silica gel column chromatography with elution system A to obtain the title compound **1c** (140 mg, yield: 76.1%).
MS m/z (ESI): 290.2 [M+1]

### Step 2

### 6-Chloro-N-(4-methoxybenzyl)-1-(4-(pyrrolidin-1-ylmethyl)benzyl)-1H-pyrazolo[3,4-d]pyrimidin-4-amine 1e

Compound **1c** (140 mg, 0.48 mmol), 1-(4-(chloromethyl)benzyl)pyrrolidine **Id** (101.34 mg, 0.48 mmol, prepared according to the method disclosed in the patent application "WO2002012224") and potassium carbonate (66.79 mg, 0.48 mmol) were dissolved in 2 mL of *N,N*-dimethylformamide. The reaction was stopped after stirring at room temperature for 16 hours. The reaction solution was concentrated under reduced pressure, and the residue was purified by silica gel column chromatography with elution system A to obtain the title compound **1e** (70 mg, yield: 31.3%).
MS m/z (ESI): 463.2 [M+1]

### Step 3

### 6-Butoxy-N-(4-methoxybenzyl)-1-(4-(pyrrolidin-1-ylmethyl)benzyl)-1H-pyrazolo[3,4-d]pyrimidin-4-amine 1f

Compound **1e** (70 mg, 0.15 mmol), sodium *n*-butoxide (0.3 mL, 0.60 mmol) and 1 mL of *n*-butanol were added to a microwave tube successively, heated to 160 °C and stirred for 1.5 hours. The reaction was stopped, and the reaction solution was concentrated under reduced pressure. The residue was purified by silica gel column chromatography with elution system A to obtain the title compound **1f** (40 mg, yield: 52.8%).
MS m/z (ESI): 501.2 [M+1]

### Step 4

### 6-Butoxy-1-(4-(pyrrolidin-1-ylmethyl)benzyl)-1H-pyrazolo[3,4-d]pyrimidin-4-amine 1

Compound **1f** (40 mg, 0.08 mmol) and 2 mL of trifluoroacetic acid were added to a reaction flask, heated to reflux, and stirred for 24 hours. The reaction was stopped, and the reaction solution was concentrated under reduced pressure and added with 1 mL of ammonia in methanol. The residue was purified by thin layer chromatography with developing solvent system A to obtain the title compound **1** (15 mg, yield: 46.0%).
MS m/z (ESI): 381.2 [M+1]
¹H NMR (400MHz, CD₃OD) 7.98 (s, 1H), 7.41 (d, 2H), 7.36 (d, 2H), 5.48 (s, 2H), 4.39 (t, 2H), 4.13 (s, 2H), 3.12-3.08 (m, 4H), 2.02-1.98 (m, 4H), 1.80-1.76 (m, 2H), 1.55-1.49 (m, 2H), 1.01 (t, 3H).

### Example 1: Preparation of the crystal form I of the dihydrochloride salt of the compound represented by formula (I)

The compound represented by formula (I) (300 mg, 0.788 mmol) was dissolved in 5 mL of a mixed solvent of ethanol and ethyl acetate (V/V=1:1), stirred until dissolved completely, and heated to 30 °C. 4M hydrogen chloride in isopropanol (0.415 mL, 1.66 mmol) was added dropwise, and the reaction solution was cooled to room temperature and stirred for 16 hours, during which a large amount of white solid precipitated. The reaction solution was filtered, and the filter cake was collected, and dried under vacuum to obtain a product (335 mg, yield: 93%).
¹H NMR (400 MHz, CD₃OD) 8.23 (s, 1 H), 7.55 (m, 2 H), 7.44 (m, 2 H), 5.55 (s, 2 H), 4.60 (t, 2 H), 4.37 (s, 2 H), 3.40 - 3.57 (m, 2 H), 3.06 - 3.24 (m, 2 H), 2.08 - 2.26 (m, 2 H), 1.91 - 2.08 (m, 2 H), 1.80 - 1.91 (m, 2 H), 1.44 - 1.62 (m, 2 H), 1.01 (t, 3 H)

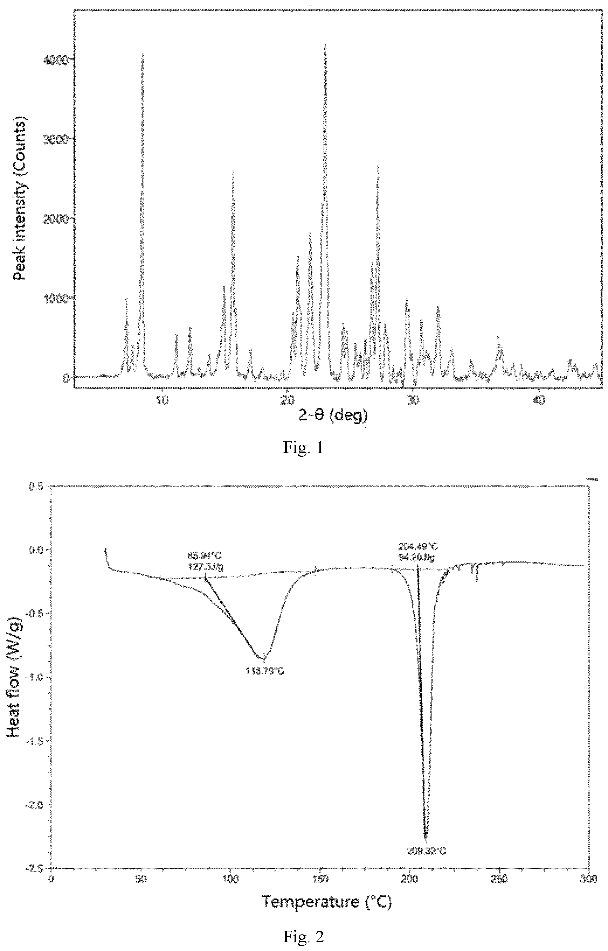

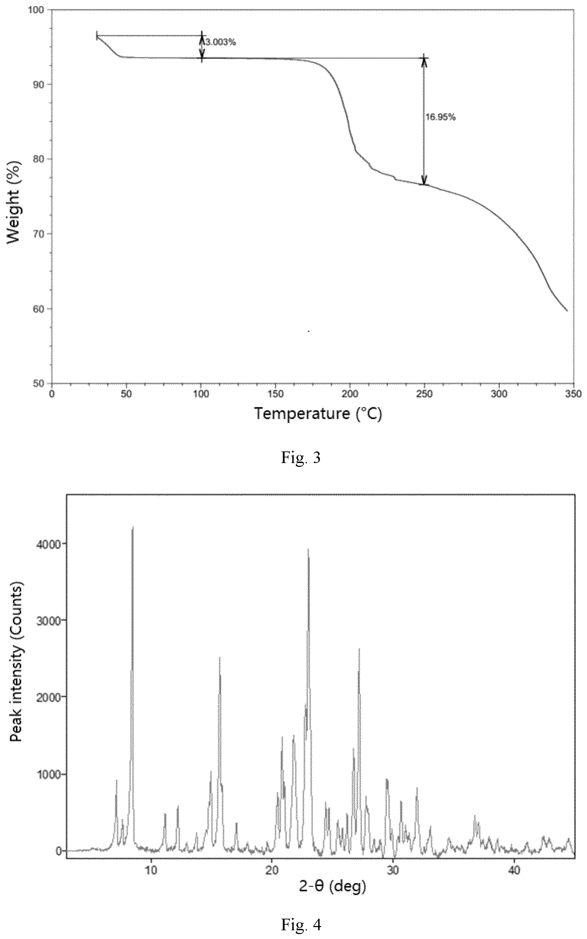

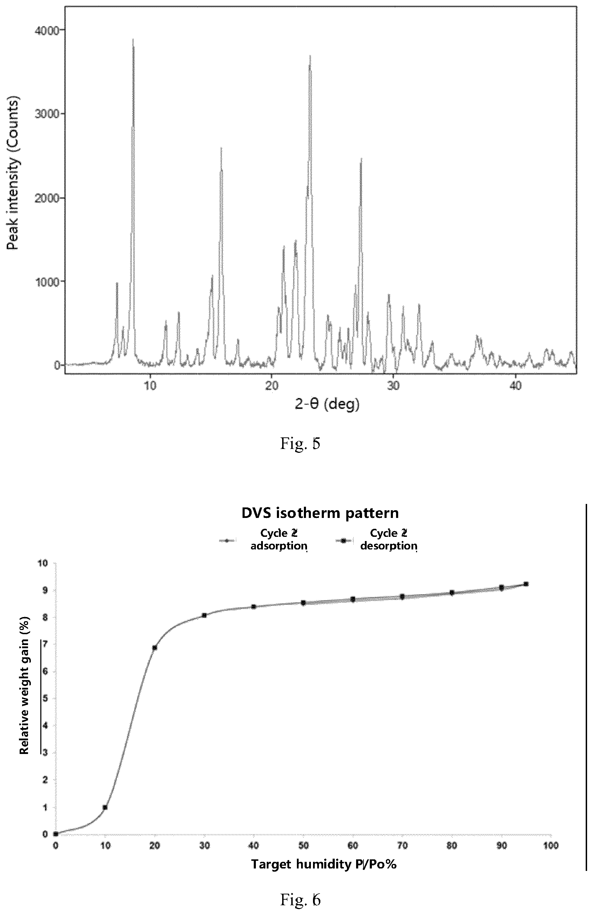

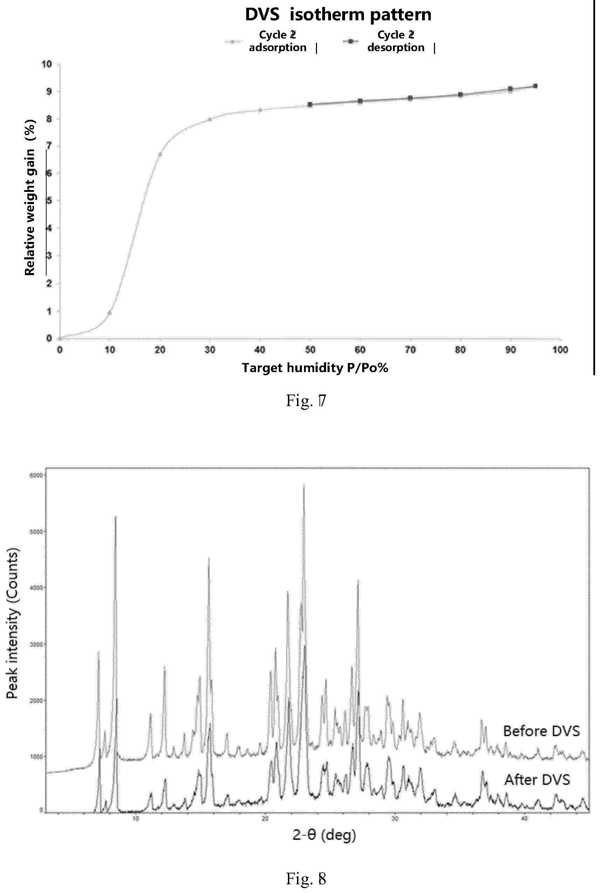

According to X-ray powder diffraction detection, the crystal form is crystal form I, and the XRPD pattern thereof is shown in Fig. 1. The DSC pattern thereof is shown in Fig. 2; the TGA pattern thereof is shown in Fig. 3; during the DSC detection process, when the temperature was raised to 150 °C, a sample was taken out and subjected to XRPD detection, the pattern is shown in Fig. 4, showing the crystal form did not change before and after the temperature rise; during the DSC detection process, when the temperature was raised to 175 °C, a sample was taken out and subjected to XRPD detection, the pattern is shown in Fig. 5, showing the crystal form did not change before and after the temperature rise; the DVS moisture absorption curves are shown in Fig. 6 and Fig. 7, and the XRPD pattern before and after the DVS detection is shown in Fig. 8, which shows the crystal form did not change.

**Table 1. characteristic peaks of the crystal form I**

| Peak No. | 2-θ (deg) | d (A) | I (%) |
|---|---|---|---|
| Peak 1 | 7.182 | 12.2990 | 26.6 |
| Peak 2 | 7.722 | 11.4390 | 5.4 |
| Peak 3 | 8.520 | 10.3700 | 79.0 |
| Peak 4 | 11.152 | 7.9280 | 16.2 |
| Peak 5 | 12.275 | 7.2040 | 16.5 |
| Peak 6 | 14.728 | 6.0099 | 10.4 |
| Peak 7 | 15.057 | 5.8790 | 22.0 |
| Peak 8 | 15.614 | 5.6708 | 49.2 |
| Peak 9 | 15.902 | 5.5680 | 18.9 |
| Peak 10 | 17.162 | 5.1620 | 7.8 |
| Peak 11 | 17.980 | 4.9300 | 7.8 |
| Peak 12 | 20.384 | 4.3530 | 12.8 |
| Peak 13 | 20.994 | 4.2281 | 42.0 |
| Peak 14 | 21.804 | 4.0728 | 40.2 |
| Peak 15 | 22.260 | 3.9900 | 6.0 |
| Peak 16 | 22.934 | 3.8745 | 100.0 |
| Peak 17 | 24.360 | 3.6510 | 12.5 |
| Peak 18 | 24.760 | 3.5930 | 8.6 |
| Peak 19 | 25.320 | 3.5150 | 11.6 |
| Peak 20 | 25.680 | 3.4661 | 9.1 |
| Peak 21 | 26.260 | 3.3910 | 13.0 |
| Peak 22 | 26.630 | 3.3450 | 19.9 |
| Peak 23 | 27.209 | 3.2747 | 47.0 |
| Peak 24 | 27.920 | 3.1930 | 17.9 |
| Peak 25 | 29.724 | 3.0031 | 21.1 |
| Peak 26 | 30.720 | 2.9080 | 18.4 |
| Peak 27 | 31.850 | 2.8072 | 6.3 |
| Peak 28 | 32.270 | 2.7720 | 8.8 |
| Peak 29 | 36.794 | 2.4407 | 10.6 |
| Peak 30 | 42.660 | 2.1175 | 4.9 |

### Example 2: Preparation of the crystal form I of the dihydrochloride salt of the compound represented by formula (I)

The compound represented by formula (I) (40 mg, 0.105 mmol) was dissolved in 0.5 mL of acetone, stirred until completely dissolved, and heated to 50 °C. 4M hydrogen chloride in isopropanol (0.055 mL, 0.22 mmol) was added dropwise, and the reaction mixture was cooled to room temperature, and stirred for 72 hours, during which a large amount of white solid precipitated. The reaction solution was filtered, the filter cake was collected, and dried under vacuum to obtain a product (20 mg, yield: 45.6%). According to X-ray powder diffraction detection, the product is the crystal form I.

### Example 3: Preparation of the crystal form II of the dihydrochloride salt of the compound represented by formula (I)

The compound represented by formula (I) (40 mg, 0.105 mmol) was dissolved in 0.5 mL of a mixed solvent of isopropanol and tetrahydrofuran (V/V = 1:1), stirred until dissolved completely, and heated to 50 °C. 4M hydrogen chloride in isopropanol (0.055 mL, 0.22 mmol) was added dropwise, and the reaction solution was cooled to room temperature and stirred for 16 hours, during which a white solid precipitated. The reaction solution was filtered, the filter cake was collected, and dried under vacuum to obtain a product (25 mg, yield: 52.5%).

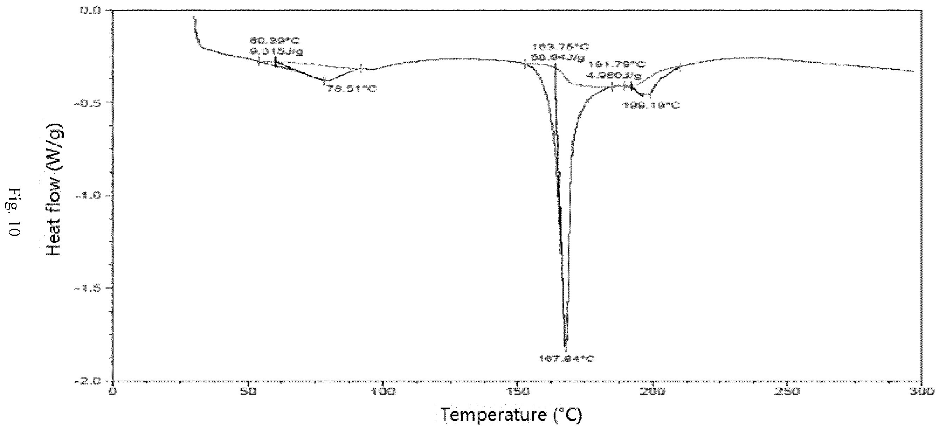

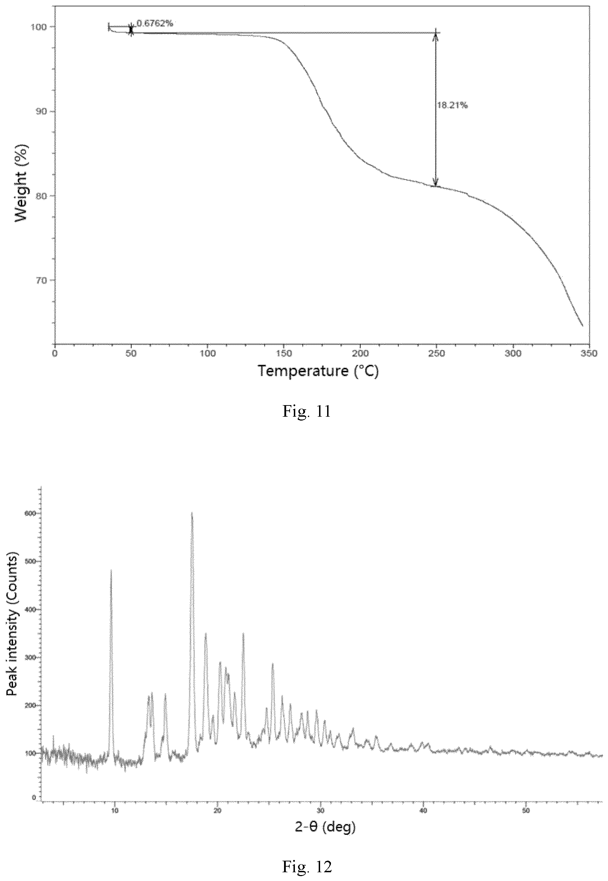

The product was defined as the crystal form II by X-ray powder diffraction detection, and the XRPD pattern is shown in Fig.. 9. The DSC pattern is shown in Fig. 10; the TGA pattern is shown in Fig. 11.

**Table 2. Characteristic peaks of the crystal form II**

| Peak No. | 2-θ (deg) | d (A) | I (%) |
|---|---|---|---|
| Peak 1 | 5.002 | 17.6519 | 5.8 |
| Peak 2 | 7.202 | 12.2638 | 3.8 |
| Peak 3 | 8.479 | 10.4200 | 12.3 |
| Peak 4 | 9.999 | 8.8392 | 93.9 |
| Peak 5 | 10.801 | 8.1845 | 32.3 |
| Peak 6 | 11.220 | 7.8796 | 4.3 |
| Peak 7 | 11.995 | 7.3719 | 9.5 |
| Peak 8 | 12.461 | 7.0974 | 20.4 |
| Peak 9 | 13.725 | 6.4466 | 12.1 |
| Peak 10 | 14.120 | 6.2673 | 17.7 |
| Peak 11 | 15.761 | 5.6182 | 56.7 |
| Peak 12 | 16.484 | 5.3732 | 10.1 |
| Peak 13 | 17.020 | 5.2051 | 39.6 |
| Peak 14 | 18.680 | 4.7462 | 61.2 |
| Peak 15 | 20.135 | 4.4064 | 16.6 |
| Peak 16 | 20.558 | 4.3167 | 45.3 |
| Peak 17 | 20.863 | 4.2543 | 48.0 |
| Peak 18 | 21.289 | 4.1702 | 9.1 |
| Peak 19 | 21.641 | 4.1030 | 32.2 |
| Peak 20 | 22.319 | 3.9800 | 9.3 |
| Peak 21 | 22.960 | 3.8702 | 22.2 |
| Peak 22 | 24.202 | 3.6744 | 25.5 |
| Peak 23 | 24.541 | 3.6244 | 100.0 |
| Peak 24 | 26.240 | 3.3935 | 65.8 |
| Peak 25 | 26.660 | 3.3409 | 44.9 |
| Peak 26 | 27.196 | 3.2763 | 6.6 |
| Peak 27 | 28.262 | 3.1551 | 26.4 |
| Peak 28 | 28.681 | 3.1100 | 37.9 |
| Peak 29 | 29.518 | 3.0236 | 8.8 |
| Peak 30 | 31.017 | 2.8808 | 9.6 |
| Peak 31 | 31.355 | 2.8506 | 9.6 |
| Peak 32 | 32.725 | 2.7343 | 5.0 |
| Peak 33 | 33.198 | 2.6964 | 13.4 |
| Peak 34 | 36.810 | 2.4397 | 6.0 |
| Peak 35 | 37.880 | 2.3732 | 5.3 |
| Peak 36 | 39.335 | 2.2887 | 4.5 |
| Peak 37 | 41.004 | 2.1993 | 4.2 |

### Example 4: Measurement of the solubility of the crystal form I of the present disclosure

The amorphous samples of the compound represented by formula (I) and the crystal form I samples of the dihydrochloride salt of the compound represented by formula (I) obtained in the present disclosure were further evaluated for solubility in PBS 7.4 and FaSSIF solutions.

### Test results

**Table 3. The solubility test results of the compound represented by formula (I) and the crystal form I of the dihydrochloride thereof**

| Sample | FasSIF | PBS 7.4 | Log D |
|---|---|---|---|
| | Solubility (mg/mL) | Solubility (mg/mL) | |
| The compound represented by formula (I) | 1.09 | 0.030 | 1.21 |
| The crystal form I (Example 1) | 1.25 | 0.050 | 1.17 |

### Example 5: Study of the hygroscopicity of the crystal form I of the dihydrochloride salt of the compound represented by formula (I)

Surface Measurement Systems advantage 2 was used, the experiment was carried out at 25 °C with humidity starting from 50%, the humidity range observed was 0%-95%, the step size was 10%, and the judgment standard was that the mass change was less than 0.01% within 10000 min, and two cycles were performed.

### Experimental results

**Table 4. The study results of the hygroscopicity of the crystal form I of the dihydrochloride salt of the compound represented by formula (I)**

| Sample for test | 0.0%RH-95.0%RH | Crystal form |
|---|---|---|
| crystal form I (Example 1) | 9.19% (with hygroscopicity) | unchanged |

### Experimental conclusion:

It can be seen from Table 4 that under the condition of 25 °C, the crystal form I sample of the compound represented by formula (I) of the present disclosure has water absorption increased as the increase of humidity between 10%RH-90.0%RH, and a weight change of 6.628%, which is less than 15% but not less than 2%, indicating the sample is slightly hygroscopic; the desorption process of the sample basically coincides with the adsorption process during the humidity change of 10%-90.0%; the DVS pattern is shown in Fig. 8, and the X-ray powder diffraction pattern comparison before and after DVS shows that the crystal form has not changed before and after DVS (see Fig. 8).

**Example 6:** the crystal form I of the dihydrochloride salt of the compound represented by formula (I) (example 1) was spread and uncovered, and the steadily of the sample was evaluated under heating (40 °C, 60 °C), light illumination (4500 Lux), and high humidity (RH 75%, RH 90%) with a period of 20 days.

### Experimental results:

**Table 5. Experimental results of influencing factors**

| The crystal form I of the dihydrochloride salt | | | | |
|---|---|---|---|---|
| Sample placement conditions | | Purity (%) | Maximum single impurity (%) | Total impurities (%) |
| 0 day | | 99.47 | 0.11 | 0.53 |
| light ill uminati on | 4 days | 99.45 | 0.14 | 0.55 |
| | 10 days | 99.54 | 0.11 | 0.46 |
| | 20 days | 99.41 | 0.12 | 0.59 |
| 40 °C | 4 days | 99.49 | 0.11 | 0.51 |
| | 10 days | 99.52 | 0.12 | 0.48 |
| | 20 days | 99.46 | 0.12 | 0.54 |
| 60 °C | 4 days | 99.44 | 0.15 | 0.56 |
| | 10 days | 99.53 | 0.11 | 0.47 |
| | 20 days | 99.42 | 0.11 | 0.58 |
| 75% RH | 4 days | 99.38 | 0.15 | 0.62 |
| | 10 days | 99.53 | 0.12 | 0.47 |
| | 20 days | 99.47 | 0.11 | 0.53 |
| 90% RH | 4 days | 99.46 | 0.11 | 0.54 |
| | 10 days | 99.54 | 0.12 | 0.46 |
| | 20 days | 99.52 | 0.11 | 0.48 |

The experimental results of the influencing factors in Table 5 show that the physical and chemical stability of the crystal form I is good under the conditions of light illumination, high temperature of 40 °C and 60 °C, light illumination, high humidity of 75% and 90%.

### Example 7. Preparation of the crystal form A of the monohydrochloride salt of the compound represented by formula (I)

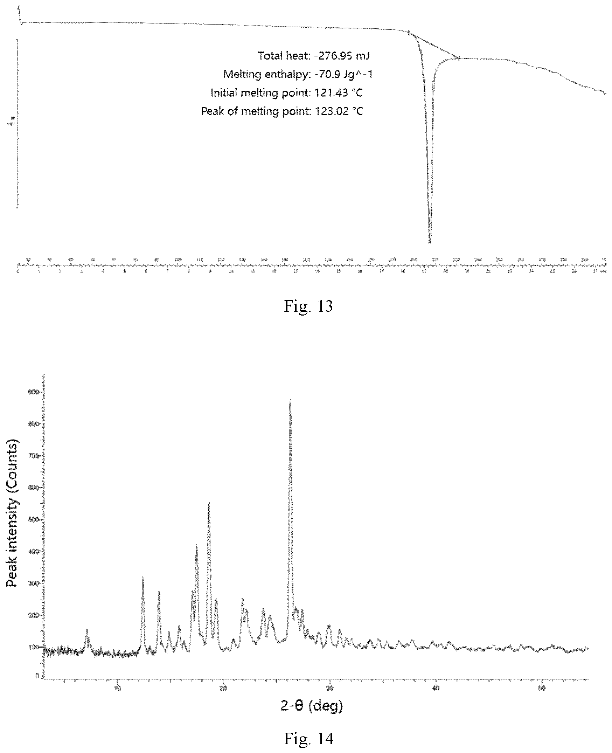

500 mg of the compound represented by formula (I) was weighed accurately, added with 12.5 mL of acetonitrile and stirred until dissolved, and then heated to 50 °C. 53.1 mg of concentrated hydrochloric acid was added quickly, and turbidity appeared immediately. The obtained mixture was maintained at 50 °C and stirred at closed state for 2 hours, cooled to room temperature naturally, and centrifuged to remove the supernatant. The obtained precipitate was dried at 50 °C. According to the results of ion chromatography, the product has a Cl⁻ number of 8.4%, which means that it contains 1 chloride ion through calculation. Through X-powder diffraction detection, the crystal form is the crystal form A, and the XRPD pattern is shown in Fig. 12. The DSC pattern is shown in Fig. 13.

**Table 6. Characteristic peaks of the crystal form A**

| Peak No. | 2-θ (deg) | d (A) | I (%) |
|---|---|---|---|
| Peak 1 | 9.647 | 9.16079 | 81.5 |
| Peak 2 | 10.324 | 8.56185 | 4.5 |
| Peak 3 | 12.323 | 7.17695 | 0.1 |
| Peak 4 | 13.018 | 6.79524 | 9.1 |
| Peak 5 | 13.306 | 6.64901 | 25.3 |
| Peak 6 | 13.644 | 6.48464 | 25.8 |
| Peak 7 | 14.633 | 6.04868 | 5.7 |
| Peak 8 | 14.936 | 5.92678 | 26.8 |
| Peak 9 | 15.655 | 5.6561 | 2.0 |
| Peak 10 | 16.943 | 5.2288 | 2.2 |
| Peak 11 | 17.533 | 5.0543 | 100.0 |
| Peak 12 | 18.365 | 4.82711 | 2.9 |
| Peak 13 | 18.866 | 4.69998 | 46.9 |
| Peak 14 | 19.553 | 4.53646 | 9.9 |
| Peak 15 | 20.261 | 4.37936 | 31.7 |
| Peak 16 | 20.836 | 4.25988 | 28.3 |
| Peak 17 | 21.038 | 4.21948 | 27.5 |
| Peak 18 | 21.684 | 4.0952 | 18.6 |
| Peak 19 | 22.515 | 3.94587 | 45.5 |
| Peak 20 | 23.030 | 3.85882 | 4.0 |
| Peak 21 | 24.007 | 3.70388 | 1.4 |
| Peak 22 | 24.451 | 3.63758 | 4.9 |
| Peak 23 | 24.775 | 3.5908 | 13.1 |
| Peak 24 | 25.396 | 3.50435 | 31.5 |
| Peak 25 | 26.006 | 3.42351 | 1.6 |
| Peak 26 | 26.306 | 3.38511 | 16.5 |
| Peak 27 | 27.095 | 3.2884 | 14.4 |
| Peak 28 | 27.694 | 3.21853 | 1.9 |
| Peak 29 | 28.182 | 3.1639 | 10.1 |
| Peak 30 | 28.742 | 3.1035 | 11.3 |
| Peak 31 | 29.621 | 3.0134 | 11.6 |
| Peak 32 | 30.388 | 2.9391 | 9.1 |
| Peak 33 | 30.982 | 2.88409 | 3.9 |
| Peak 34 | 31.604 | 2.82873 | 2.0 |
| Peak 35 | 31.870 | 2.80568 | 3.1 |
| Peak 36 | 32.848 | 2.7244 | 3.1 |
| Peak 37 | 33.203 | 2.69604 | 5.6 |
| Peak 38 | 34.536 | 2.59499 | 0.8 |
| Peak 39 | 35.380 | 2.53499 | 3.8 |
| Peak 40 | 36.757 | 2.4431 | 0.9 |
| Peak 41 | 38.757 | 2.32156 | 0.2 |
| Peak 42 | 39.867 | 2.2594 | 1.9 |
| Peak 43 | 40.445 | 2.22846 | 1.9 |

### Example 8. Preparation of the crystal form B of the monohydrochloride salt of the compound represented by formula (I)

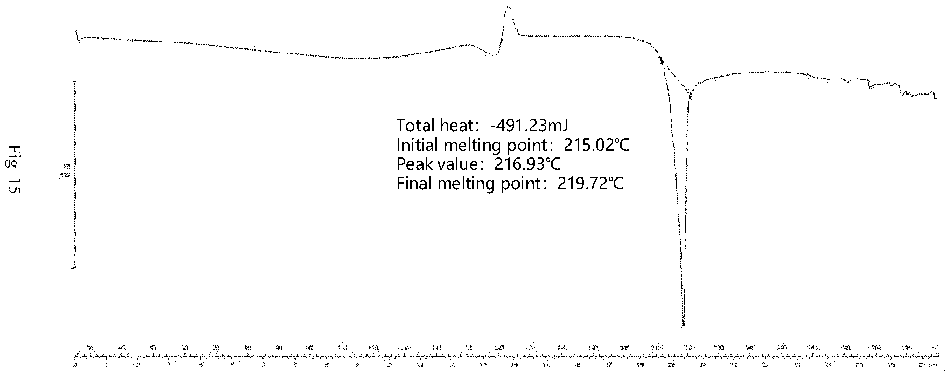

500 mg of the compound represented by formula (I) was weighed accurately, added with 12.5 mL of ethyl acetate and stirred until dissolved, and then heated to 50 °C. 53.1 mg of concentrated hydrochloric acid was added quickly, and turbidity appeared immediately. The obtained mixture was maintained at 50 °C and stirred at closed state for 2 hours, cooled to room temperature naturally, and centrifuged to remove the supernatant. The obtained precipitate was dried at 50 °C. According to the results of ion chromatography, the product has a Cl⁻ number of 8.4%, which means that it contains 1 chloride ion through calculation. Through X-powder diffraction detection, the crystal form is the crystal form B, and the XRPD pattern is shown in Fig. 14. The DSC pattern is shown in Fig. 15.

**Table 7. Characteristic peaks of the crystal form B**

| Peak No. | 2-θ (deg) | d (A) | I (%) |
|---|---|---|---|
| Peak 1 | 3.226 | 27.36576 | 4.0 |
| Peak 2 | 7.094 | 12.45104 | 8.2 |
| Peak 3 | 7.362 | 11.99874 | 6.3 |
| Peak 4 | 8.503 | 10.39114 | 1.9 |
| Peak 5 | 12.421 | 7.12027 | 32.2 |
| Peak 6 | 13.065 | 6.7707 | 3.1 |
| Peak 7 | 13.937 | 6.34928 | 25.2 |
| Peak 8 | 14.900 | 5.941 | 7.8 |
| Peak 9 | 15.837 | 5.59146 | 9.8 |
| Peak 10 | 16.280 | 5.44031 | 3.3 |
| Peak 11 | 17.095 | 5.18272 | 23.6 |
| Peak 12 | 17.492 | 5.06591 | 42.7 |
| Peak 13 | 17.910 | 4.9487 | 6.3 |
| Peak 14 | 18.647 | 4.75467 | 59.6 |
| Peak 15 | 19.317 | 4.59131 | 19.5 |
| Peak 16 | 20.227 | 4.38665 | 0.4 |
| Peak 17 | 20.895 | 4.24798 | 3.3 |
| Peak 18 | 21.823 | 4.06935 | 19.5 |
| Peak 19 | 22.183 | 4.00415 | 14.1 |
| Peak 20 | 22.522 | 3.9446 | 4.1 |
| Peak 21 | 23.315 | 3.81224 | 1.5 |
| Peak 22 | 23.777 | 3.73915 | 13.3 |
| Peak 23 | 24.391 | 3.64636 | 10.1 |
| Peak 24 | 24.650 | 3.60868 | 5.6 |
| Peak 25 | 26.321 | 3.38327 | 100.0 |
| Peak 26 | 26.857 | 3.31699 | 11.7 |
| Peak 27 | 27.432 | 3.24866 | 11.7 |
| Peak 28 | 27.895 | 3.19584 | 4.3 |
| Peak 29 | 28.405 | 3.13956 | 1.4 |
| Peak 30 | 28.963 | 3.08036 | 4.6 |
| Peak 31 | 29.918 | 2.98414 | 7.9 |
| Peak 32 | 30.946 | 2.88732 | 5.7 |
| Peak 33 | 31.618 | 2.82749 | 2.6 |
| Peak 34 | 32.119 | 2.78454 | 1.6 |
| Peak 35 | 32.786 | 2.72935 | 0.0 |
| Peak 36 | 33.830 | 2.64754 | 1.9 |
| Peak 37 | 34.622 | 2.58871 | 2.1 |
| Peak 38 | 35.373 | 2.53545 | 1.2 |
| Peak 39 | 36.500 | 2.45974 | 2.2 |
| Peak 40 | 37.209 | 2.41446 | 0.0 |
| Peak 41 | 37.877 | 2.37342 | 2.1 |
| Peak 42 | 39.755 | 2.26554 | 1.6 |
| Peak 43 | 40.506 | 2.22525 | 0.8 |

### Example 9. Preparation of the crystal form C of the monohydrochloride salt of the compound represented by formula (I)

500 mg of the compound represented by formula (I) was weighed accurately, added with 12.5 mL of 1,4-dioxane and stirred until dissolved, and then heated to 50 °C. 53.1 mg of concentrated hydrochloric acid was added quickly, and turbidity appeared immediately. The obtained mixture was maintained at 50 °C and stirred at closed state for 2 hours, cooled to room temperature naturally, and centrifuged to remove the supernatant. The obtained precipitate was dried at 50 °C.

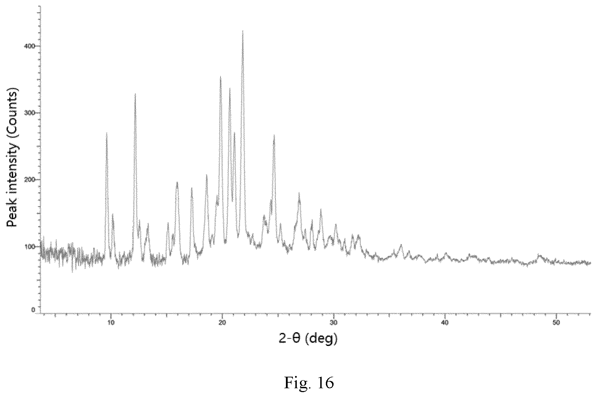

According to the results of ion chromatography, the product has a Cl⁻ number of 8.4%, which means that it contains 1 chloride ion through calculation. Through X-powder diffraction detection, the crystal form is the crystal form C, and the XRPD pattern is shown in Fig. 16.

**Table 8. Characteristic peaks of the crystal form C**

| Peak No. | 2-θ (deg) | d (A) | I (%) |
|---|---|---|---|
| Peak 1 | 6.175 | 14.30133 | 6.4 |
| Peak 2 | 8.509 | 10.38289 | 5.8 |
| Peak 3 | 9.641 | 9.16633 | 58.7 |
| Peak 4 | 10.199 | 8.66596 | 19.2 |
| Peak 5 | 11.165 | 7.91818 | 3.1 |
| Peak 6 | 12.176 | 7.26334 | 79.8 |
| Peak 7 | 12.542 | 7.05221 | 17.2 |
| Peak 8 | 13.302 | 6.65053 | 15.0 |
| Peak 9 | 15.118 | 5.85574 | 16.4 |
| Peak 10 | 15.592 | 5.67864 | 11.2 |
| Peak 11 | 15.950 | 5.55199 | 36.1 |
| Peak 12 | 16.840 | 5.26065 | 3.2 |
| Peak 13 | 17.288 | 5.12537 | 32.6 |
| Peak 14 | 18.579 | 4.77181 | 35.0 |
| Peak 15 | 19.093 | 4.6445 | 4.1 |
| Peak 16 | 19.547 | 4.53771 | 19.3 |
| Peak 17 | 19.859 | 4.46709 | 77.8 |
| Peak 18 | 20.675 | 4.29261 | 71.6 |
| Peak 19 | 21.083 | 4.21055 | 49.7 |
| Peak 20 | 21.838 | 4.06656 | 100.0 |
| Peak 21 | 22.394 | 3.96696 | 1.8 |
| Peak 22 | 22.715 | 3.91146 | 2.2 |
| Peak 23 | 23.795 | 3.73639 | 13.1 |
| Peak 24 | 23.963 | 3.71057 | 9.3 |
| Peak 25 | 24.628 | 3.61191 | 48.6 |
| Peak 26 | 25.222 | 3.52816 | 10.4 |
| Peak 27 | 25.653 | 3.46979 | 0.4 |
| Peak 28 | 26.914 | 3.31005 | 23.5 |
| Peak 29 | 27.424 | 3.24964 | 5.7 |
| Peak 30 | 28.068 | 3.17653 | 10.3 |
| Peak 31 | 28.886 | 3.08843 | 15.4 |
| Peak 32 | 29.678 | 3.0078 | 3.8 |
| Peak 33 | 30.179 | 2.95897 | 10.4 |
| Peak 34 | 30.523 | 2.92641 | 2.8 |
| Peak 35 | 30.965 | 2.88558 | 2.8 |
| Peak 36 | 31.730 | 2.81776 | 6.3 |
| Peak 37 | 32.213 | 2.77661 | 5.9 |
| Peak 38 | 32.937 | 2.71718 | 2.0 |
| Peak 39 | 35.433 | 2.53135 | 1.5 |
| Peak 40 | 36.036 | 2.49032 | 3.7 |

**Example 10:** the crystal form A (example 7) of the monohydrochloride salt of the compound of formula (I) was spread and uncovered, and the stability of the sample was evaluated under heating (40 °C, 60 °C), light illumination (4500 Lux), high humidity (RH 75%, RH 90%) conditions with a period of 20 days.

### Experimental results:

**Table 9. Experimental results of influencing factors**

| Sample placement conditions | | Purity (%) | Maximum single impurity (%) | Total impurities (%) |
|---|---|---|---|---|
| 0 day | | 99.74 | 0.09 | 0.26 |
| | 4 days | 99.70 | 0.09 | 0.30 |
| Light ill uminati on | 10 days | 99.75 | 0.06 | 0.25 |
| | 20 days | 99.63 | 0.08 | 0.37 |
| 40 °C | 4 days | 99.72 | 0.09 | 0.28 |
| | 10 days | 99.72 | 0.06 | 0.28 |
| | 20 days | 99.70 | 0.06 | 0.30 |
| 60 °C | 4 days | 99.72 | 0.08 | 0.28 |
| | 10 days | 99.75 | 0.06 | 0.25 |
| | 20 days | 99.70 | 0.06 | 0.30 |
| 75% RH | 4 days | 99.72 | 0.09 | 0.28 |
| | 10 days | 99.74 | 0.06 | 0.26 |
| | 20 days | 99.68 | 0.08 | 0.32 |
| 90%RH | 4 days | 99.72 | 0.08 | 0.28 |
| | 10 days | 99.76 | 0.06 | 0.24 |
| | 20 days | 99.72 | 0.08 | 0.28 |

The results show that the crystal form A of the monohydrochloride salt has good chemical stability under the above conditions, and there is no significant increase in impurities.

**Example 11:** the crystal form B of the monohydrochloride salt of the compound represented by formula (I) (example 8) was spread and uncovered, and the stability of the sample was evaluated under heating (40 °C, 60 °C), light illumination (4500 Lux), high humidity (RH 75%, RH 90%) conditions with a period of 20 days.

### Experimental results:

**Table 10. Experimental results of influencing factors**

| Sample placement conditions | | Purity (%) | Maximum single impurity (%) | Total impurities (%) |
|---|---|---|---|---|
| 0 day | | 99.54 | 0.10 | 0.46 |
| Light ill uminati on | 4 days | 99.51 | 0.10 | 0.49 |
| | 10 days | 99.56 | 0.10 | 0.44 |
| | 20 days | 99.45 | 0.11 | 0.55 |
| 40°C | 4 days | 99.31 | 0.19 | 0.69 |
| | 10 days | 99.53 | 0.10 | 0.47 |
| | 20 days | 99.39 | 0.14 | 0.61 |
| 60°C | 4 days | 99.34 | 0.14 | 0.66 |
| | 10 days | 99.42 | 0.10 | 0.58 |
| | 20 days | 99.21 | 0.10 | 0.79 |
| 75%RH | 4 days | 99.54 | 0.11 | 0.46 |
| | 10 days | 99.61 | 0.10 | 0.39 |
| | 20 days | 99.57 | 0.10 | 0.43 |
| 90%RH | 4 days | 99.54 | 0.10 | 0.46 |
| | 10 days | 99.61 | 0.10 | 0.39 |
| | 20 days | 99.59 | 0.10 | 0.41 |

The results show that the crystal form B of monohydrochloride has good chemical stability under the above conditions, and there is no significant increase in impurities.

**Example 12:** the crystal form C of the monohydrochloride salt of the compound represented by formula (I) (example 9) was spread and uncovered, and the stability of the sample was evaluated under heating (40 °C, 60 °C), light illumination (4500 Lux), high humidity (RH 75%, RH 90%) conditions with a period of 20 days.

### Experimental results:

**Table 11. Experimental results of influencing factors**

| Sample placement conditions | | Purity (%) | Maximum single impurity (%) | Total impurities (%) |
|---|---|---|---|---|
| 0 day | | 99.55 | 0.10 | 0.45 |
| Light ill uminati on | 4 days | 99.57 | 0.11 | 0.43 |
| | 10 days | 99.64 | 0.09 | 0.36 |
| | 20 days | 99.54 | 0.10 | 0.46 |
| 40 °C | 4 days | 99.55 | 0.10 | 0.45 |
| | 10 days | 99.60 | 0.10 | 0.40 |
| | 20 days | 99.53 | 0.10 | 0.47 |
| 60 °C | 4 days | 99.50 | 0.10 | 0.50 |
| | 10 days | 99.58 | 0.10 | 0.42 |
| | 20 days | 99.42 | 0.13 | 0.58 |
| 75% RH | 4 days | 99.55 | 0.11 | 0.45 |
| | 10 days | 99.61 | 0.09 | 0.39 |
| | 20 days | 99.57 | 0.10 | 0.43 |
| 90% RH | 4 days | 99.51 | 0.11 | 0.49 |
| | 10 days | 99.61 | 0.10 | 0.39 |
| | 20 days | 99.58 | 0.10 | 0.42 |

The results show that the crystal form C of the monohydrochloride salt has good chemical stability under the above conditions, and there is no significant increase in impurities.

**Example 13:** Three batches of the crystal form I of the dihydrochloride salt of the compound of formula (I) were subjected to a long-term stability investigation of 9 months under the conditions of 25 °C±2 °C, 60%RH±5%RH The results are shown in Table 12.

Table 12. Investigation of long-term stability of the crystal form I of the dihydrochloride salt of the compound of formula (I)

| Sample | Placement conditions | | Purity% | Purity % | Purity % | Crystal form |
|---|---|---|---|---|---|---|
| | | Initial | 3 months | 3 months | 9 months | |
| Batch 1 | 25 °C, 60%RH | 99.57% | 99.52% | 99.57% | 99.50% | I |
| Batch 2 | 25 °C, 60%RH | 99.53% | 99.50% | 99.48% | 99.48% | I |
| Batch 3 | 25 °C, 60%RH | 99.44% | 99.38% | 99.33% | 99.40% | I |

The long-term stability test results in Table 12 show that the crystal form I of the dihydrochloride salt of the compound of formula (I) has good physical and chemical stability under stability condition of 25 °C, 60% RH for 9 months.

### Test example:

### Biological evaluation

### Test example 1: Determination of the agonistic effect of the compound represented by formula (I) on human TLR7

The agonistic effect of the compound represented by formula (I) on the hTLR7 protein expressed in HEK-Blue™ hTLR7 stably transfected cells was determined by the following experimental method:

### I. Experimental Materials and Instruments

1. DMEM (Gibco, 10564-029),
2. Fetal bovine serum (GIBCO, 10099),
3. Penicillin-Streptomycin (Gibco, 15140-122),
4. Normocin (Invivogen,ant-nr-1),
5. Blasticindin (Invivogen, ant-bl-1),
6. Zeocin (Invivogen, ant-zn-1),
7. Flexstation 3 multi-function microplate reader (Molecµlar Devices),
8. HEK-Blue™ hTLR7 cell line (InvivoGen, hkb-hTLR7),
9. HEK-Blue detection reagent (InvivoGen, hb-det3),

### II. Experimental Procedures

A bag of HEK-Blue detection dry powder was dissolved in 50 mL of water free of endotoxin, and the solution was then placed in an incubator at 37 °C for 10 minutes followed by sterile filtration to prepare a HEK-Blue detection medium. The compound was firstly formulated into a 20 mM stock solution, then diluted with pure DMSO to a maximum concentration of 6×10⁶ nM, and a total of 10 points were obtained by a 3-fold gradient dilution.

The above formulated compound was firstly diluted 20-fold with the medium, then 20 µL of the diluted compound was added to each well. The supernatant was removed from the HEK-Blue™ hTLR7 cells, to which 2-5 mL of pre-warmed PBS was then added. The cells were placed in an incubator for 1-2 minutes, gently pipetted, and counted by trypan blue staining. The cells were re-suspended in the HEK-Blue detection medium, and the concentration was adjusted to 2.2×10⁵ cells/mL. 180 µL of cells was added to the above 96-well plate added with 20 µL of the compound, and incubated at 37 °C for 6-16 hours.

The plate was read with a microplate reader at a wavelength of 620 nm. The corresponding OD values were obtained, and the EC₅₀ value of the compound was calculated by Graphpad Prism.

The agonistic effect of the compound represented by formula (I) on human TLR7 was determined by the above test, and the measured EC₅₀ value was 28 nM.

Conclusion: the compound represented by formula (I) has a significant agonistic effect on human TLR7.

### Test example 2: Determination of the agonistic effect of the compound represented by formula (I) on human TLR8

The agonistic effect of the compound represented by formula (I) on the hTLR8 protein expressed in HEK-Blue™ hTLR8 stably transfected cells was determined by the following experimental method:

### I. Experimental Materials and Instruments

1. DMEM (Gibco, 10564-029),
2. Fetal bovine serum (GIBCO, 10099),
3. Penicillin-Streptomycin (Gibco, 15140-122),
4. Normocin (Invivogen, ant-nr-1),
5. Blasticindin (Invivogen, ant-bl-1),
6. Zeocin (Invivogen, ant-zn-1),
7. Flexstation 3 multi-function microplate reader (Molecµlar Devices),
8. HEK-Blue™ hTLR8 cell line (InvivoGen, hkb-hTLR7),
9. HEK-Blue detection reagent (InvivoGen, hb-det3),

### II. Experimental Procedures

A bag of HEK-Blue detection dry powder was dissolved in 50 mL of water free of endotoxin, and the solution was then placed in an incubator at 37 °C for 10 minutes followed by sterile filtration to prepare a HEK-Blue detection medium. The compound was firstly formulated into a 20 mM stock solution, then diluted with pure DMSO to a maximum concentration of 6×10⁶ nM, and a total of 10 points were obtained by a 3-fold gradient dilution. The compound was firstly diluted 20-fold with the medium, then 20 µL of the diluted compound was added to each well.

The supernatant was removed from the HEK-Blue™ hTLR8 cells, to which 2-5 mL of pre-warmed PBS was then added. The cells were placed in an incubator for 1-2 minutes, gently pipetted, and counted by trypan blue staining. The cells were re-suspended in the HEK-Blue detection medium and the concentration was adjusted to 2.2×10⁵ cells/mL. 180 µL of cells was added to the above 96-well plate added with 20 µL of the compound, and incubated at 37 °C for 6-16 hours.

The plate was read with a microplate reader at a wavelength of 620 nm. The corresponding OD values were obtained, and the EC₅₀ value of the compound was calculated by Graphpad Prism.

The agonistic effect of the compound represented by formula (I) on human TLR8 was determined by the above test, and the measured EC₅₀ value was >30000 nM, Emax 8%.

Conclusion: the compound represented by formula (I) has no agonistic effect on human TLR8, indicating that the compound represented by formula (I) has high selectivity for TLR7.

### Test example 3: Determination of the ability of the compound of the present disclosure to stimulate the secretion of IFN-α from peripheral blood mononuclear cells (PBMC)

The ability of the compound of the present disclosure to stimulate the secretion of IFN-α from PBMC was determined by the following experimental method:

### I. Experimental Materials and Instruments

1.RPMI 1640 (Invitrogen, 11875),
2. FBS (Gibco, 10099-141),
3. Penicillin-Streptomycin (Gibco, 15140-122),
4. Ficoll-Paque PREMIUM (GE, 17-5442-02),
5. Trypan blue solution (Sigma, T8154-100ML),
6. SepMate™-50 (Stemcell, 15460),
7. Bright-Line™ Blood Cell Counter (Sigma, Z359629-1EA)
8. 96-well flat bottom plate (Corning, 3599),
9. 96-well v bottom plate (Corning, 3894),
10. Human IFN-α kit (cisbio, 6FHIFPEB),
11. PHERAStar Multi-Function Microplate Reader (BMG, PHERAStar).

### II. Experimental Procedures

The compound was diluted with pure DMSO to a maximum concentration of 5 mM, and a total of 9 points were obtained by a 4-fold gradient dilution. 4 µL of the compound was then added to 196 µL of RMPI 1640 medium containing 10% FBS and mixed well. 50 µL of the mixture was taken from each well and added to a new 96-well cell culture plate.

All reagents were equilibrated to room temperature. 60 mL of blood and PBS+2% FBS were added to a 250 mL culture flask, gently pipetted, mixed well and diluted. 15 mL of lymphocyte separation solution Ficoll-Paque PREMIUM and then 30 mL of diluted blood were added to a 50 mL PBMC centrifuge tube SepMateTM-50. The mixture was centrifuged at 1200 g for 10 minutes at room temperature. The supernatant was taken and then centrifuged at 300 g for 8 minutes. The cells were re-suspended in the RMPI 1640 medium containing 10% FBS and counted, and the number of PBMCs was adjusted to 3.33×10⁶ cells/mL. 150 µL of the cell solution was added to the plate added with the compound, and incubated in an incubator at 37 °C, 5.0% CO₂ for 24 hours.

The cell culture plate was placed in a centrifuge, and centrifuged at 1200 rpm for 10 minutes at room temperature. 150 µL of the supernatant was taken from each well. The reagents in the human IFN-α kit were firstly equilibrated to normal temperature. The anti-IFN-α-Eu³⁺-Cryptate conjugate and the anti-IFN-α-d2-conjugate were formulated in the dark according to the kit instructions, and both of them were mixed well with the conjugate buffer at a ratio of 1:40. 16 µL of the supernatant obtained by centrifugation was then added to each well. 2 µL of the prepared anti-IFN-α-Eu³⁺-Cryptate conjugate and anti-IFN-α-d2-conjugate were then added to each well, mixed well by shaking. The cells were incubated in the dark at room temperature for 3 hours.

The plate was read with PHERAStar in the HTRF mode. The lowest compound concentration that stimulate cytokine level of at least 3 times higher than the minimum detection limit was defined as the minimal effective concentration (MEC) value of the compound in the cytokine stimulation test.

The ability of the compound represented by formula (I) to stimulate the secretion of IFN-α from PBMC was determined by the above test, and the measured MEC value was 6 nM.

Conclusion: based on the data of the activity of stimulating the secretion of IFN-α from PBMC, it can be seen the compound represented by formula (I) has the advantage of lower effective concentration.

### Test example 4: Inhibitory effect of the compound represented by formula (I) on the enzyme activity of midazolam metabolite site of CYP3A4 in human liver microsome

The effect of the compound represented by formula (I) on the enzyme activity of midazolam metabolite site of CYP3A4 in human liver microsome was determined by the following experimental method:

### I. Experimental Materials and Instruments

1. Phosphate buffer (PB S),
2. NADPH (Sigma N-1630),
3. Human liver microsomes (Corning Gentest),
4. ABI QTrap 4000 liquid chromatograph/mass spectrometer (AB Sciex),
5. Inertsil C8-3 column, 4.6×50 mm, 5 µm (Dikma Technologies Inc., USA),
6. CYP probe substrate (midazolam/10µM) and positive control inhibitor (ketoconazole).

### II. Experimental Procedures

100 mM PBS buffer was formulated, which was then used to formulate 2.5 mg/mL microsome solution and 5 mM NADPH solution. The 5X concentration of the compound working solution was diluted with PBS gradient (150, 50, 15, 5, 1.5, 0.15, 0.015, 0 µM). The 5X concentration of ketoconazole working solution was diluted with PBS gradient (150, 50, 15, 5, 1.5, 0.15, 0.015, 0 µM). Dextromethorphan working solution was diluted with PBS to a concentration of 50 µM.

20 µL of the 2.5 mg/mL microsome solution, 20 µL of the 50 µM testosterone working solution, 20 µL of MgCl₂ solution and 20 µL of the compound working solution (150, 50, 15, 5, 1.5, 0.15, 0.015, 0 µM, different reaction systems for each concentration) were taken respectively and mixed well. For the positive control group, the compound was replaced with the same concentration of ketoconazole. The mixture together with the 5 mM NADPH solution was pre-incubated at 37 °C for 5 minutes. After 5 minutes, 20 µL of NADPH was added to each well, the reaction was started and incubated for 30 minutes. All the incubated samples were present in duplicate. After 30 minutes, 250 µL of acetonitrile containing internal standard was added to all samples, mixed well, shaken at 800 rpm for 10 minutes, and then centrifuged at 3700 rpm for 10 minutes. 80 µL of the supernatant was taken and analyzed by LC-MS/MS.

The data was calculated by Graphpad Prism to obtain the ICso value of the compound on the midazolam metabolite site of CYP3A4.

The compound represented by formula (I) has no inhibitory effect on the midazolam metabolic site of CYP3A4 in human liver microsome, the measured IC₅₀ value was 14 µM.

Conclusion: the compound represented by formula (I) has no inhibitory effect on the midazolam metabolic site of CYP3A4 in human liver microsome, and shows better safety, suggesting that metabolic drug interactions based on the midazolam metabolism site of CYP3A4 will not occur.

### Test example 5: Inhibitory effect of the compound represented by formula (I) on the enzyme activity of CYP2D6 in human liver microsome

The effect of the compound represented by formula (I) on the enzyme activity of CYP2D6 in human liver microsome was determined by the following experimental method:

### I. Experimental Materials and Instruments

1. Phosphate buffer (PB S),
2. NADPH (Sigma N-1630),
3. Human liver microsomes (Corning Gentest),
4. ABI QTrap 4000 liquid chromatograph/mass spectrometer (AB Sciex),
5. Inertsil C8-3 column, 4.6×50 mm, 5 µm (Dikma Technologies Inc., USA),
6. CYP probe substrate (dextromethorphan/10 µM), and positive control inhibitor (quinidine).

### II. Experimental Procedures

100 mM PBS buffer was formulated, which was then used to formulate 2.5 mg/mL microsome solution and 5 mM NADPH solution. The 5X concentration of the compound working solution was diluted with PBS gradient (150, 50, 15, 5, 1.5, 0.15, 0.015, 0 µM). The 5X concentration of quinidine working solution was diluted with PBS gradient (150, 50, 15, 5, 1.5, 0.15, 0.015, 0 µM). Dextromethorphan working solution was diluted with PBS to a concentration of 50 µM.

20 µL of the 2.5 mg/mL microsome solution, 20 µL of the 50 µM testosterone working solution, 20 µL of MgCl₂ solution and 20 µL of the compound working solution (150, 50, 15, 5, 1.5, 0.15, 0.015, 0 µM, different reaction systems for each concentration) were taken respectively and mixed well. For the positive control group, the compound was replaced with the same concentration of quinidine. The mixture together with the 5 mM NADPH solution was pre-incubated at 37 °C for 5 minutes. After 5 minutes, 20 µL of NADPH was added to each well, the reaction was started and incubated for 30 minutes. All the incubated samples were present in duplicate. After 30 minutes, 250 µL of acetonitrile containing internal standard was added to all samples, mixed well, shaken at 800 rpm for 10 minutes, and then centrifuged at 3700 rpm for 10 minutes. 80 µL of the supernatant was taken and analyzed by LC-MS/MS.

The data were calculated by Graphpad Prism to obtain the ICso value of the compound on the metabolite site of CYP2D6.

The compound represented by formula (I) has no inhibitory effect against CYP2D6, the measured IC₅₀ value was more than 30 µM.

Conclusion: the compound represented by formula (I) has no inhibitory effect on the enzyme activity of CYP2D6 in human liver microsome, suggesting that the metabolic drug interaction based on CYP2D6 will not occur.

### Test example 6: Inhibitory effect of the compound represented by formula (I) on the enzyme activity of testosterone metabolite site of CYP3A4 in human liver microsome

The effect of the compound represented by formula (I) on the enzyme activity of testosterone metabolite site of CYP3A4 in human liver microsome was determined by the following experimental method:

### I. Experimental Materials and Instruments

1. Phosphate buffer (PBS),
2. NADPH (Sigma N-1630),
3. Human liver microsomes (Corning Gentest),
4. ABI QTrap 4000 liquid chromatograph/mass spectrometer (AB Sciex),
5. Inertsil C8-3 column, 4.6×50 mm, 5 µm (Dikma Technologies Inc., USA),
6. CYP probe substrate (testosterone/100 µM), and positive control inhibitor (ketoconazole).

### II. Experimental Procedures

100 mM PBS buffer was formulated, which was then used to formulate 2.5 mg/mL microsome solution and 5 mM NADPH solution. The 5X concentration of the compound working solution was diluted with PBS gradient (150, 50, 15, 5, 1.5, 0.15, 0.015, 0 µM). The 5X concentration of ketoconazole working solution was diluted with PBS gradient (150, 50, 15, 5, 1.5, 0.15, 0.015, 0 µM). Dextromethorphan working solution was diluted with PBS to a concentration of 50 µM.

20 µL of the 2.5 mg/mL microsome solution, 20 µL of the 50 µM testosterone working solution, 20 µL of MgCl₂ solution and 20 µL of the compound working solution (150, 50, 15, 5, 1.5, 0.15, 0.015, 0 µM, different reaction systems for each concentration) were taken respectively and mixed well. For the positive control group, the compound was replaced with the same concentration of ketoconazole. The mixture together with the 5 mM NADPH solution was pre-incubated at 37 °C for 5 minutes. After 5 minutes, 20 µL of NADPH was added to each well, the reaction was started and incubated for 30 minutes. All the incubated samples were present in duplicate. After 30 minutes, 250 µL of acetonitrile containing internal standard was added to all samples, mixed well, shaken at 800 rpm for 10 minutes, and then centrifuged at 3700 rpm for 10 minutes. 80 µL of the supernatant was taken and analyzed by LC-MS/MS.

The data was calculated by Graphpad Prism to obtain the ICso value of the compound on the testosterone metabolite site of CYP3A4.

The measured ICso value of the compound represented by formula (I) (example 1) on the testosterone metabolite site of CYP3A4 in human liver microsome was 4 µM.

Conclusion: the compound represented by formula (I) has weak inhibitory effect on the testosterone metabolite site of CYP3A4 in human liver microsome, and shows better safety.

## Claims

1. A hydrochloride salt of the compound represented by formula (I):

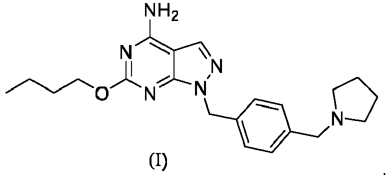

2. The hydrochloride salt of the compound represented by formula (I) as defined in claim 1, wherein the hydrochloride salt is dihydrochloride salt or monohydrochloride salt.

3. A crystal form I of a dihydrochloride salt of the compound represented by formula (I), wherein the X-ray powder diffraction pattern thereof has characteristic peaks at 2θ angles of 7.182, 8.520, 12.275, 15.057, 15.614, 20.994, 21.804, and 22.934,

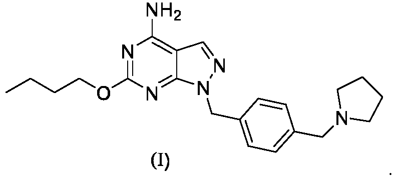

4. The crystal form I of the dihydrochloride salt of the compound represented by formula (I) as defined in claim 3, wherein the X-ray powder diffraction pattern thereof has characteristic peaks at 2θ angles of 7.182, 8.520, 11.152, 12.275, 15.057, 15.614, 15.902, 17.162, 20.384, 20.994, 21.804, 22.934, 24.360, 26.260, 26.630, 27.209, and 29.724.

5. The crystal form I of the dihydrochloride salt of the compound represented by formula (I) as defined in claim 4, wherein the X-ray powder diffraction pattern thereof has characteristic peaks at 2θ angles of 7.182, 7.722, 8.520, 11.152, 12.275, 15.057, 15.614, 15.902, 17.162, 20.384, 20.994, 21.804, 22.934, 24.360, 25.320, 26.260, 26.630, 27.209, 27.920, 29.724, 30.720, and 32.270.

6. A crystal form II of a dihydrochloride salt of the compound represented by formula (I), wherein the X-ray powder diffraction pattern thereof has characteristic peaks at 2θ angles of 8.479, 9.999, 10.801, 12.461, 13.725, 14.120, 15.761, 17.020, 18.680, 20.135, 20.558, 20.863, 21.641, 22.960, 24.202, 24.541, 26.240, 26.660, 28.262, and 28.681,

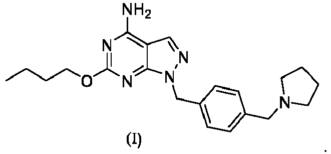

7. The crystal form II of the dihydrochloride salt of the compound represented by formula (I) as defined in claim 6, wherein the X-ray powder diffraction pattern thereof has characteristic peaks at 2θ angles of 5.002, 7.202, 8.479, 9.999, 10.801, 11.220, 11.995, 12.461, 13.725, 14.120, 15.761, 16.484, 17.020, 18.680, 20.135, 20.558, 20.863, 21.289, 21.641, 22.319, 22.960, 24.202, 24.541, 26.240, 26.660, 27.196, 28.262, 28.681, 29.518, 31.017, 31.355, 32.725, 33.198, 36.810, 37.880, 39.335, and 41.004.

8. A crystal form A of a monohydrochloride salt of the compound represented by formula (I), wherein the X-ray powder diffraction pattern thereof has characteristic peaks at 2θ angles of 9.647, 13.306, 13.644, 14.936, 17.533, 18.866, 20.261, and 22.515,

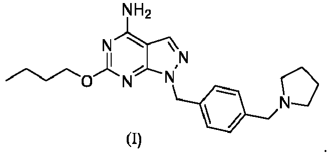

9. The crystal form A of the monohydrochloride salt of the compound represented by formula (I) as defined in claim 8, wherein the X-ray powder diffraction pattern thereof has characteristic peaks at 2θ angles of 9.647, 13.018, 13.306, 13.644, 14.936, 17.533, 18.866, 20.261, 20.836, 21.038, 21.684, 22.515, 24.775, 25.396, 26.306, 27.095, 28.182, 28.742, 29.621, and 30.388.

10. A crystal form B of a monohydrochloride salt of the compound represented by formula (I), wherein the X-ray powder diffraction pattern thereof has characteristic peaks at 2θ angles of 12.421, 13.937, 17.095, 17.492, 18.647, 19.317, 21.823, 22.183, and 26.321,

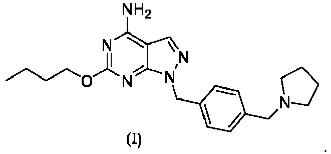

11. The crystal form B of the monohydrochloride salt of the compound represented by formula (I) as defined in claim 10, wherein the X-ray powder diffraction pattern thereof has characteristic peaks at 2θ angles of 7.094, 12.421, 13.937, 14.900, 15.837, 17.095, 17.492, 18.647, 19.317, 21.823, 22.183, 23.777, 24.391, 26.321, 26.857, 27.432, 29.918, and 30.946.

12. A crystal form C of a monohydrochloride salt of the compound represented by formula (I), wherein the X-ray powder diffraction pattern thereof has characteristic peaks at 2θ angles of 9.641, 10.199, 12.176, 15.950, 17.288, 18.579, 19.859, 20.675, 21.083, 21.838 and 24.628,

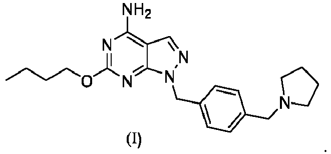

13. The crystal form C of the monohydrochloride salt of the compound represented by formula (I) as defined in claim 12, wherein the X-ray powder diffraction pattern thereof has characteristic peaks at 2θ angles of 9.641, 10.199, 12.176, 12.542, 13.302, 15.118, 15.592, 15.950, 17.288, 18.579, 19.547, 19.859, 20.675, 21.083, 21.838, 23.795, 23.963, 24.628, 25.222, 26.914, 28.068, 28.886, and 30.179.

14. The crystal form of the hydrochloride of the compound represented by formula (I) as defined in any one of claims 3-13, wherein the error ranges of the 2θ angles are ±0.2.

15. A preparation method of the hydrochloride salt of the compound represented by formula (I) as defined in claim 1, comprising a step of salifying the compound represented by formula (I) with hydrochloric acid.

16. A preparation method of the crystal form I of the dihydrochloride salt of the compound represented by formula (I) as defined in any one of claims 3-5 and 14, selected from:
a method i: placing the compound represented by formula (I) in a solvent for crystallization, clarifying, adding hydrochloric acid, crystallizing, filtering, and drying to obtain the target crystal form I; or
a method ii: placing the dihydrochloride salt of the compound represented by formula (I) in a solvent for crystallization, crystallizing, filtering, and drying to obtain the target crystal form I, wherein the crystallizing method is selected from crystallizing at room temperature, crystallizing by cooling, crystallizing by volatilizing solvent, or crystallizing by adding a seed crystal to induce crystallization;
in the method i or the method ii, the solvent for crystallization does not include a mixed solvent of isopropanol-tetrahydrofuran;
in the method i or the method ii, the solvent for crystallization is one or more selected from ether solvents, alcohol solvents, ester solvents, ketone solvents, nitrile solvents, and halogenated hydrocarbon solvents;
in the method i or the method ii, the ether solvent is selected from tetrahydrofuran, diethyl ether, propylene glycol monomethyl ether, methyl *tert*-butyl ether, isopropyl ether or 1,4-dioxane;
in the method i or the method ii, the alcohol solvent is selected from methanol, ethanol, isopropanol, *n*-propanol, isopentanol or trifluoroethanol;
in the method i or the method ii, the ester solvent is selected from ethyl acetate, isopropyl acetate or butyl acetate;
in the method i or the method ii, the ketone solvent is selected from acetone, acetophenone, isobutyl methyl ketone or methyl pyrrolidone;
in the method i or the method ii, the nitrile solvent is selected from acetonitrile, propionitrile; the halogenated hydrocarbon solvent is selected from chloromethane, dichloromethane, chloroform or carbon tetrachloride;
in the method i or the method ii, the amount of the hydrochloric acid is 2--30 times, preferably 2-15 times, and most preferably 2-5 times the amount of substance of the compound represented by formula (I).

17. The preparation method as defined in claim 16, wherein, in the method i or the method ii, the solvent for crystallization is selected from tetrahydrofuran, isopropyl ether, 1,4-dioxane, methanol, ethanol, isopropanol, ethyl acetate, isopropyl acetate, acetone, acetonitrile, dichloromethane, isopropanol-isopropyl acetate, isopropanol-isopropyl ether, isopropanol-dioxane, ethanol-dioxane, ethanol-tetrahydrofuran, ethanol-isopropyl ether, ethanol-isopropyl acetate, ethanol-acetonitrile, isopropanol-acetonitrile, methanol-isopropyl ether, methanol-isopropyl acetate, methanol-acetonitrile, dichloromethane-tetrahydrofuran, isopropanol-tetrahydrofuran, isopropanol-ethyl acetate or methanol-ethyl acetate.

18. A preparation method of the crystal form II of the dihydrochloride salt of the compound of formula (I) as defined in any one of claims 6, 7 and 14, placing the compound of formula (I) in a solvent for crystallization, clarifying, adding hydrochloric acid, crystallizing, filtering, and drying to obtain the target crystal form II, the solvent for crystallization is a mixed solvent of isopropanol-tetrahydrofuran; the amount of the hydrochloric acid is 2-30 times, preferably 2-15 times, most preferably 2-5 times the amount of substance of the compound represented by formula (I).

19. A preparation method of the crystal form A of the monohydrochloride salt of the compound represented by formula (I) as defined in any one of claims 8, 9, and 14, selected from:
a method i: placing the compound represented by formula (I) in a solvent for crystallization, clarifying, adding hydrochloric acid, crystallizing, filtering, and drying to obtain the target crystal form A; or
a method ii: placing the monohydrochloride salt of the compound represented by formula (I) in a solvent for crystallization, crystallizing, filtering, and drying to obtain the target crystal form A, wherein the crystallizing method is selected from crystallizing at room temperature, crystallizing by cooling, crystallizing by volatilizing solvent, or crystallizing by adding a seed crystal to induce crystallization;
in the method i or the method ii, the solvent for crystallization is at least one selected from nitrile solvents and ketone solvents;
in the method i or the method ii, the ketone solvent is selected from acetone, acetophenone, methyl isobutyl ketone or methyl pyrrolidone, preferably acetone;
in the method i or the method ii, the nitrile solvent is selected from acetonitrile or propionitrile, preferably acetonitrile;
in the method i or the method ii, the amount of the hydrochloric acid is 1-2 times the amount of substance of the compound represented by formula (I).

20. A preparation method of the crystal form B of the monohydrochloride salt of the compound represented by formula (I) as defined in any one of claims 10, 11 and 14, selected from:
a method i: placing the compound represented by formula (I) in a solvent for crystallization, clarifying, adding hydrochloric acid, crystallizing, filtering, and drying to obtain the target crystal form B; or
a method ii: placing the monohydrochloride salt of the compound represented by formula (I) in a solvent for crystallization, crystallizing, filtering, and drying to obtain the target crystal form B, wherein the crystallizing method is selected from crystallizing at room temperature, crystallizing by cooling, crystallizing by volatilizing solvent, or crystallizing by adding a seed crystal to induce crystallization;
in the method i or the method ii, the solvent for crystallization is selected from ester solvents, the ester solvent is selected from ethyl acetate, isopropyl acetate or butyl acetate, preferably ethyl acetate;
in the method i or the method ii, the amount of the hydrochloric acid is 1-2 times the amount of substance of the compound represented by formula (I).

21. A preparation method of the crystal form C of the monohydrochloride salt of the compound represented by formula (I) as defined in any one of claims 12-14, selected from:
a method i: placing the compound represented by formula (I) in a solvent for crystallization, clarifying, adding hydrochloric acid, crystallizing, filtering, and drying to obtain the target crystal form C; or
a method ii: placing the monohydrochloride salt of the compound represented by formula (I) in a solvent for crystallization, crystallizing, filtering, and drying to obtain the target crystal form C, wherein the crystallizing method is selected from crystallizing at room temperature, crystallizing by cooling, crystallizing by volatilizing solvent, or crystallizing by adding a seed crystal to induce crystallization;
in the method i or the method ii, the solvent for crystallization is selected from ether solvents, tetrahydrofuran, diethyl ether, propylene glycol monomethyl ether, methyl tert-butyl ether, isopropyl ether or 1,4-dioxane, preferably 1,4-dioxane;
in the method i or the method ii, the amount of the hydrochloric acid is 1-2 times the amount of substance of the compound represented by formula (I).

22. A pharmaceutical composition, comprising the following components:
i) at least one of the hydrochloride salt of the compound represented by formula (I) as defined in claim 1 or 2, the crystal form I of the dihydrochloride salt of the compound represented by formula (I) as defined in any one of claims 3-5 and 14, the crystal form II of the dihydrochloride salt of the compound represented by formula (I) as defined in any one of claims 6, 7 and 14, the crystal form A of the monohydrochloride salt of the compound represented by formula (I) as defined in any one of claims 8, 9 and 14, the crystal form B of the monohydrochloride salt of the compound represented by formula (I) as defined in any one of claims 10, 11 and 14, and the crystal form C of the monohydrochloride salt of the compound represented by formula (I) as defined any one of claims 12-14; and
ii) one or more of pharmaceutically acceptable carriers, diluents or excipients.

23. A preparation method of the pharmaceutical composition as defined in claim 22, wherein the preparation method comprises a step of mixing the components.

24. Use of the hydrochloride salt of the compound represented by formula (I) as defined in claim 1 or 2, the crystal form I of the dihydrochloride salt of the compound represented by formula (I) as defined in any one of claims 3-5 and 14, the crystal form II of the dihydrochloride salt of the compound represented by formula (I) as defined in any one of claims 6, 7 and 14, the crystal form A of the monohydrochloride salt of the compound represented by formula (I) as defined in any one of claims 8, 9 and 14, the crystal form B of the monohydrochloride salt of the compound represented by formula (I) as defined in any one of claims 10, 11 and 14, or the crystal form C of the monohydrochloride salt of the compound represented by formula (I) as defined in any one of claims 12-14 in the manufacture of a medicament for treating viral infection caused by virus, wherein the virus is selected from dengue virus, flavivirus, West Nile virus, Japanese encephalitis virus, tick-borne encephalitis virus, Kunjin virus, Murray Valley encephalitis virus, Saint Louis encephalitis virus, Omsk hemorrhagic fever virus, bovine viral diarrhea virus, Zika virus, HIV, HBV, HCV, HPV, RSV, SARS and/or influenza virus.

25. Use of the hydrochloride salt of the compound represented by formula (I) as defined in claim 1 or 2, the crystal form I of the dihydrochloride salt of the compound represented by formula (I) as defined in any one of claims 3-5 and 14, the crystal form II of the dihydrochloride salt of the compound represented by formula (I) as defined in any one of claims 6, 7 and 14, the crystal form A of the monohydrochloride salt of the compound represented by formula (I) as defined in any one of claims 8, 9 and 14, the crystal form B of the monohydrochloride salt of the compound represented by formula (I) as defined in any one of claims 10, 11 and 14, or the crystal form C of the monohydrochloride salt of the compound represented by formula (I) as defined any one of claims 12-14 in the manufacture of a medicament for treating or preventing melanoma, non-small cell lung cancer, hepatocellular carcinoma, basal cell carcinoma, renal cell carcinoma, bladder cancer, myeloma, allergic rhinitis, asthma, COPD, ulcerative colitis and/or hepatic fibrosis.
